# EUROPEAN PATENT SPECIFICATION

(11) **EP 1 718 648 B1**
(45) Date of publication and mention of the grant of the patent: **23.04.2008**
(21) Application number: 05708021.0
(22) Date of filing: 16.02.2005
(51) Int. Cl.: C07D 471/14, C07D 471/04, A61K 31/435

(54) **TRICYCLIC IMIDAZOPYRIDINES AND INTERMEDIATES FOR THE SYNTHESIS THEREOF**
TRICYCLISCHE IMIDAZOPYRIDINE UND ZWISCHENPRODUKTE FÜR DEREN SYNTHESE
IMIDAZOLPYRIDINES TRICYCLIQUES ET INTERMEDIAIRES POUR LEUR SYNTHESE

(30) Priority: 17.02.2004 EP 04003467; 09.06.2004 EP 04102627; 21.12.2004 EP 04106802
(43) Date of publication of application: 08.11.2006
(73) Proprietor: Nycomed GmbH, 78467 Konstanz (DE)
(72) Inventor: CHIESA, M. Vittoria, 78464 Konstanz (DE); BUHR, Wilm, 78465 Konstanz (DE); ZIMMERMANN, Peter Jan, 71139 Ehningen (DE); BREHM, Christof, 68163 Mannheim (DE); PALMER, Andreas, 78224 Singen (DE); KROMER, Wolfgang, 78464 Konstanz (DE); POSTIUS, Stefan, 78467 Konstanz (DE); SIMON, Wolfgang-Alexander, 78464 Konstanz (DE)
(74) Representative: Wolf, Ulrich
(86) International application number: PCT/EP2005/050667
(87) International publication number: WO 2005/077949

(56) References cited:
- WO-A-01/72754
- WO-A-98/42707
- WO-A-03/014123
- US-A1- 2002 169 320
- US-B1- 6 503 923

## Description

### Technical field

The invention relates to a process for the preparation of tricyclic imidazopyridines, to valuable intermediates used in said process, to new tricyclic imidazopyridines produced with that process and their use in the pharmaceutical industry as active compounds for preparing medicaments.

### Prior Art

U.S. Patent 4,468,400 describes tricyclic imidazo[1,2-a]pyridines having different ring systems fused to the imidazopyridine skeleton, which compounds are said to be suitable for treating peptide ulcer disorders. The International Patent Applications WO98/42707, WO98/54188, WO00/17200, WO00/26217, WO 00/63211, WO 01/72756, WO 01/72754, WO 01/72755, WO 01/72757, WO 02/34749, WO 03/014120, WO 03/016310, WO 03/014123, WO 03/068774 and WO 03/091253 disclose tricyclic imidazopyridine derivatives having a very specific substitution pattern, which compounds are likewise said to be suitable for treating gastrointestinal disorders.

### Description of the Invention

It has now been found that the compounds disclosed for example in WO 03/014123 with X = NH, which were not described by way of example in said patent application, can be prepared with a broad variety of substituents by way of a general reaction sequence starting from novel intermediates.

The invention thus relates in a first aspect to compounds of the formula 1, 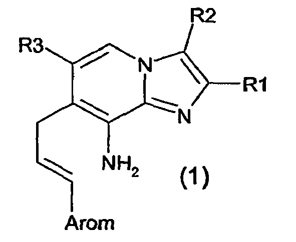 where
- R1: is hydrogen, 1-4C-alkyl, 3-7C-cycloalkyl, 3-7C-cycloalkyl-1-4C-alkyl, 1-4C-alkoxy-1-4C-alkyl, 1-4C-alkoxycarbonyl, 2-4C-alkenyl, fluoro-1-4C-alkyl or hydroxy-1-4C-alkyl,
- R2: is hydrogen, 1-4C-alkyl, 3-7C-cycloalkyl, 3-7C-cycloalkyl-1-4C-alkyl, 1-4C-alkoxycarbonyl, hydroxy-1-4C-alkyl, halogen, 2-4C-alkenyl, 2-4C-alkynyl, fluoro-1-4C-alkyl or cyanomethyl,
- R3: is halogen, hydroxy-1-4C-alkyl, 1-4C-alkoxy-1-4C-alkyl, 1-4C-alkoxy-1-4C-alkoxy-1-4C-alkyl, 1-4C-alkoxycarbonyl, fluoro-1-4C-alkoxy-1-4C-alkyl or the radical -CO-NR31 R32,
where
R31 is hydrogen, 1-7C-alkyl, hydroxy-1-4C-alkyl, 3-7C-cycloalkyl, 1-4C-alkoxy-1-4C-alkyl and
R32 is hydrogen, 1-7C-alkyl, hydroxy-1-4C-alkyl, 3-7C-cycloalkyl or 1-4C-alkoxy-1-4C-alkyl,
or where
R31 and R32 together and including the nitrogen atom to which they are attached form a pyrrolidino, piperidino or morpholino radical
- Arom: is a R4-, R5-, R6- and R7-substituted mono- or bicyclic aromatic radical selected from the group consisting of phenyl, naphthyl, pyrrolyl, pyrazolyl, imidazolyl, 1,2,3-triazolyl, indolyl, benzimidazolyl, furanyl (furyl), benzofuranyl (benzofuryl), thiophenyl (thienyl), benzothiophenyl (benzothienyl), thiazolyl, isoxazolyl, pyridinyl, pyrimidinyl, quinolinyl and isoquinolinyl,
where
R4 is hydrogen, 1-4C-alkyl, hydroxy-1-4C-alkyl, 1-4C-alkoxy, 2-4C-alkenyloxy, carboxyl, 1-4C-alkoxycarbonyl, carboxy-1-4C-alkyl, halogen, hydroxyl, aryl, aryl-1-4C-alkyl, aryloxy, aryl-1-4C-alkoxy, trifluoromethyl, nitro, amino, mono- or di-1-4C-alkylamino or sulfonyl,
R5 is hydrogen, 1-4C-alkyl, 1-4C-alkoxy, 1-4C-alkoxycarbonyl, halogen, trifluoromethyl or hydroxyl,
R6 is hydrogen, 1-4C-alkyl or halogen and
R7 is hydrogen, 1-4C-alkyl or halogen, where
aryl is phenyl or substituted phenyl having one, two or three identical or different substituents from the group consisting of 1-4C-alkyl, 1-4C-alkoxy, carboxyl, halogen, trifluoromethyl, nitro, trifluoromethoxy, hydroxyl and cyano,
and their salts.

1-4C-Alkyl denotes straight-chain or branched alkyl radicals having 1 to 4 carbon atoms. Examples which may be mentioned are the butyl, isobutyl, sec-butyl, tert-butyl, propyl, isopropyl, ethyl and methyl radicals.

3-7C-Cycloalkyl denotes cyclopropyl, cyclobutyl, cyclopentyl, cyclohexyl and cycloheptyl, among which cyclopropyl, cyclobutyl and cyclopentyl are preferred.

3-7C-Cycloalkyl-1-4C-alkyl denotes one of the abovementioned 1-4C-alkyl radicals which is substituted by one of the abovementioned 3-7C-cycloalkyl radicals. Examples which may be mentioned are the cyclopropylmethyl, the cyclohexylmethyl and the cyclohexylethyl radicals.

1-4C-Alkoxy denotes radicals which, in addition to the oxygen atom, contain a straight-chain or branched alkyl radical having 1 to 4 carbon atoms. Examples which may be mentioned are the butoxy, isobutoxy, sec-butoxy, tert-butoxy, propoxy, isopropoxy and preferably the ethoxy and methoxy radicals.

1-4C-Alkoxy-1-4C-alkyl denotes one of the abovementioned 1-4C-alkyl radicals which is substituted by one of the abovementioned 1-4C-alkoxy radicals. Examples which may be mentioned are the methoxymethyl, the methoxyethyl and the butoxyethyl radicals.

1-4C-Alkoxycarbonyl (-CO-1-4C-alkoxy) denotes a carbonyl group to which is attached one of the abovementioned 1-4C-alkoxy radicals. Examples which may be mentioned are the methoxycarbonyl (CH₃O-C(O)-) and the ethoxycarbonyl (CH₃CH₂O-C(O)-) radicals.

2-4C-Alkenyl denotes straight-chain or branched alkenyl radicals having 2 to 4 carbon atoms. Examples which may be mentioned are the 2-butenyl, 3-butenyl, 1-propenyl and the 2-propenyl (allyl) radicals.

2-4C-Alkynyl denotes straight-chain or branched alkynyl radicals having 2 to 4 carbon atoms. Examples which may be mentioned are the 2-butynyl, the 3-butynyl, the 2-propynyl (propargyl) and, preferably, the 1-ethynyl, 1-propynyl and 1-butynyl radicals.

Fluoro-1-4C-alkyl denotes one of the abovementioned 1-4C-alkyl radicals which is substituted by one or more fluorine atoms. An example which may be mentioned is the trifluoromethyl radical.

Hydroxy-1-4C-alkyl denotes abovementioned 1-4C-alkyl radicals which are substituted by a hydroxyl group. Examples which may be mentioned are the hydroxymethyl, the 2-hydroxyethyl and the 3-hydroxypropyl radicals.

For the purpose of the invention, halogen is bromine, chlorine and fluorine.

1-4C-Alkoxy-1-4C-alkoxy denotes one of the abovementioned 1-4C-alkoxy radicals which is substituted by a further 1-4C-alkoxy radical. Examples which may be mentioned are the radicals 2-(methoxy)ethoxy (CH₃-O-CH₂-CH₂-O-) and 2-(ethoxy)ethoxy (CH₃-CH₂-O-CH₂-CH₂-O-).

1-4C-Alkoxy-1-4C-alkoxy-1-4C-alkyl denotes one of the abovementioned 1-4C-alkoxy-1-4C-alkyl radicals which is substituted by one of the abovementioned 1-4C-alkoxy radicals. An example which may be mentioned is the radical 2-(methoxy)ethoxymethyl (CH₃-O-CH₂-CH₂-O-CH₂-).

Fluoro-1-4C-alkoxy-1-4C-alkyl denotes one of the abovementioned 1-4C-alkyl radicals which is substituted by a fluoro-1-4C-alkoxy radical. Here, fluoro-1-4C-alkoxy denotes one of the abovementioned 1-4C-alkoxy radicals which is fully or predominantly substituted by fluorine. Examples of fully or predominantly fluorine-substituted 1-4C-alkoxy which may be mentioned are the 1,1,1,3,3,3-hexafluoro-2-propoxy, the 2-trifluoromethyl-2-propoxy, the 1,1,1-trifluoro-2-propoxy, the perfluoro-tert-butoxy, the 2,2,3,3,4,4,4-heptafluoro-1-butoxy, the 4,4,4-trifluoro-1-butoxy, the 2,2,3,3,3-pentafluoropropoxy, the perfluoroethoxy, the 1,2,2-trifluoroethoxy, in particular the 1,1,2,2-tetrafluoroethoxy, the 2,2,2-trifluoroethoxy, the trifluoromethoxy and preferably the difluoromethoxy radicals.

1-7C-Alkyl denotes straight-chain or branched alkyl radicals having 1 to 7 carbon atoms. Examples which may be mentioned are the heptyl, isoheptyl-(5-methylhexyl), hexyl, isohexyl-(4-methylpentyl), neohexyl-(3,3-dimethylbutyl), pentyl, isopentyl-(3-methylbutyl), neopentyl-(2,2-dimethylpropyl), butyl, isobutyl, sec-butyl, tert-butyl, propyl, isopropyl, ethyl and methyl radicals.

Carboxy-1-4C-alkyl denotes, for example, the carboxymethyl (-CH₂COOH) or the carboxyethyl (-CH₂CH₂COOH) radical.

Di-1-4C-alkylamino denotes an amino radical which is substituted by two identical or different of the abovementioned 1-4C-alkyl radicals. Examples which may be mentioned are the dimethylamino, the diethylamino and the diisopropylamino radicals.

2-4C-Alkenyloxy denotes a radical which, in addition to the oxygen atom, contains a 2-4C-alkenyl radical. An example which may be mentioned is the allyloxy radical.

Aryl-1-4C-alkyl denotes an aryl-substituted 1-4C-alkyl radical. An example which may be mentioned is the benzyl radical.

Aryl-1-4C-alkoxy denotes an aryl-substituted 1-4C-alkoxy radical. An example which may be mentioned is the benzyloxy radical.

Mono- or di-1-4C-alkylamino radicals contain, in addition to the nitrogen atom, one or two of the abovementioned 1-4C-alkyl radicals. Preference is given to di-1-4C-alkylamino and in particular to dimethyl-, diethyl- or diisopropylamino.

Radicals Arom which may be mentioned are, for example, the following substituents: 4-acetoxyphenyl, 4-acetamidophenyl, 2-methoxyphenyl, 3-methoxyphenyl, 4-methoxyphenyl, 3-benzyloxyphenyl, 4-benzyloxyphenyl, 3-benzyloxy-4-methoxyphenyl, 4-benzyloxy-3-methoxyphenyl, 3,5-bis-(trifluoromethyl)phenyl, 4-butoxyphenyl, 2-chlorophenyl, 3-chlorophenyl, 4-chlorophenyl, 2-chloro-6-fluorophenyl, 3-chloro-4-fluorophenyl, 2-chloro-5-nitrophenyl, 4-chloro-3-nitrophenyl, 3-(4-chlorophenoxy)phenyl, 2,4-dichlorophenyl, 3,4-difluorophenyl, 2,4-dihydroxyphenyl, 2,6-dimethoxyphenyl, 3,4-dimethoxy-5-hydroxyphenyl, 2,5-dimethylphenyl, 3-ethoxy-4-hydroxyphenyl, 2-fluorophenyl, 4-fluorophenyl, 4-hydroxyphenyl, 2-hydroxy-5-nitrophenyl, 3-methoxy-2-nitrophenyl, 3-nitrophenyl, 2,3,5-trichlorophenyl, 2,4,6-trihydroxyphenyl, 2,3,4-trimethoxyphenyl, 2-hydroxy-1-naphthyl, 2-methoxy-1-naphthyl, 4-methoxy-1-naphthyl, 1-methyl-2-pyrrolyl, 2-pyrrolyl, 3,4-dimethyl-2-pyrrolyl, 5-ethoxycarbonyl-2,4-dimethyl-3-pyrrolyl, 3,4-dibromo-5-methyl-2-pyrrolyl, 2,5-dimethyl-1-phenyl-3-pyrrolyl, 5-carboxy-3-ethyl-4-methyl-2-pyrrolyl, 3,5-dimethyl-2-pyrrolyl, 2,5-dimethyl-1-(4-trifluoromethylphenyl)-3-pyrrolyl, 1-(2,6-dichloro-4-trifluoromethylphenyl)-2-pyrrolyl, 1-(2-nitrobenzyl)-2-pyrrolyl, 1-(2-fluorophenyl)-2-pyrrolyl, 1-(4-trifluoromethoxyphenyl)-2-pyrrolyl, 1-(4-ethoxycarbonyl)-2,5-dimethyl-3-pyrrolyl, 5-chloro-1 ,3-dimethyl-4-pyrazolyl, 5-chloro-1-methyl-3-trifluoromethyl-4-pyrazolyl, 1-(4-chlorobenzyl)-5-pyrazolyl, 1,3-dimethyl-5-(4-chlorophenoxy)-4-pyrazolyl, 1-methyl-3-trifluoromethyl-5-(3-trifluoromethylphenoxy)-4-pyrazolyl, 5-allyloxy-1-methyl-3-trifluoromethyl-4-pyrazolyl, 5-chloro-1-phenyl-3-trifluoromethyl-4-pyrazolyl, 3,5-dimethyl-1-phenyl-4-imidazolyl, 4-bromo-1-methyl-5-imidazolyl, 2-butylimidazolyl, 1-phenyl-1,2,3-triazol-4-yl, 3-indolyl, 4-indolyl, 7-indolyl, 5-methoxy-3-indolyl, 5-benzyloxy-3-indolyl, 1-benzyl-3-indolyl, 2-(4-chlorophenyl)-3-indolyl, 7-benzyloxy-3-indolyl, 6-benzyloxy-3-indolyl, 2-methyl-5-nitro-3-indolyl, 4,5,6,7-tetrafluoro-3-indolyl, 1-(3,5-difluorobenzyl)-3-indolyl, 1-methyl-2-(4-trifluorophenoxy)-3-indolyl, 1-methyl-2-benzimidazolyl, 5-nitro-2-furyl, 5-hydroxymethyl-2-furyl, 2-furyl, 3-furyl, 5-(2-nitro-4-trifluoromethylphenyl)-2-furyl, 4-ethoxycarbonyl-5-methyl-2-furyl, 5-(2-trifluoromethoxyphenyl)-2-furyl, 5-(4-methoxy-2-nitrophenyl)-2-furyl, 4-bromo-2-furyl, 5-dimethylamino-2-furyl, 5-bromo-2-furyl, 5-sulfo-2-furyl, 2-benzofuryl, 2-thienyl, 3-thienyl, 3-methyl-2-thienyl, 4-bromo-2-thienyl, 5-bromo-2-thienyl, 5-nitro-2-thienyl, 5-methyl-2-thienyl, 5-(4-methoxyphenyl)-2-thienyl, 4-methyl-2-thienyl, 3-phenoxy-2-thienyl, 5-carboxy-2-thienyl, 2,5-dichloro-3-thienyl, 2-benzothienyl, 3-methyl-2-benzothienyl, 2-bromo-5-chloro-3-benzothienyl, 2-thiazolyl, 2-amino-4-chloro-5-thiazolyl, 2,4-dichloro-5-thiazolyl, 2-diethylamino-5-thiazolyl, 3-methyl-4-nitro-5-isoxazolyl, 2-pyridyl, 3-pyridyl, 4-pyridyl, 6-methyl-2-pyridyl, 3-hydroxy-5-hydroxymethyl-2-methyl-4-pyridyl, 2,6-dichloro-4-pyridyl, 3-chloro-5-trifluoromethyl-2-pyridyl, 4-(4-chlorophenyl)-3-pyridyl, 2-chloro-5-methoxycarbonyl-6-methyl-4-phenyl-3-pyridyl, 2-chloro-3-pyridyl, 6-(3-trifluoromethylphenoxy)-3-pyridyl, 2-(4-chlorophenoxy)-3-pyridyl, 2,4-dimethoxy-5-pyrimidine, 2-quinolinyl, 3-quinolinyl, 4-quinolinyl, 2-chloro-3-quinolinyl, 2-chloro-6-methoxy-3-quinolinyl, 8-hydroxy-2-quinolinyl and 4-isoquinolinyl.

One aspect (aspect a) of the invention relates to compounds of the formula 1, in which
- R3: is halogen, hydroxy-1-4C-alkyl, 1-4C-alkoxy-1-4C-alkyl, 1-4C-alkoxy-1-4C-alkoxy-1-4C-alkyl, 1-4C-alkoxycarbonyl, fluoro-1-4C-alkoxy-1-4C-alkyl or the radical -CO-NR31 R32,
where
R31 is hydrogen, 1-7C-alkyl, hydroxy-1-4C-alkyl or 1-4C-alkoxy-1-4C-alkyl and
R32 is hydrogen, 1-7C-alkyl, hydroxy-1-4C-alkyl or 1-4C-alkoxy-1-4C-alkyl,
or where
R31 and R32 together and including the nitrogen atom to which they are attached form a pyrrolidino, piperidino or morpholino radical
and R1, R2 and Arom have the meanings as indicated in the outset.

Another aspect (aspect b) of the invention relates to compounds of the formula 1, in which
- R3: is the radical -CO-NR31 R32,
R31 is 3-7C-cycloalkyl and
R32 is hydrogen, 1-7C-alkyl, hydroxy-1-4C-alkyl, 3-7C-cycloalkyl or 1-4C-alkoxy-1-4C-alkyl,
and R1, R2 and Arom have the meanings as indicated in the outset.

The invention further relates to compounds of the formula 1, in which
- R1: is hydrogen, 1-4C-alkyl, 3-7C-cycloalkyl, 3-7C-cycloalkyl-1-4C-alkyl, 1-4C-alkoxy-1-4C-alkyl, 1-4C-alkoxycarbonyl, 2-4C-alkenyl, fluoro-1-4C-alkyl or hydroxy-1-4C-alkyl,
- R2: is hydrogen, 1-4C-alkyl, 3-7C-cycloalkyl, 3-7C-cycloalkyl-1-4C-alkyl, 1-4C-alkoxycarbonyl, hydroxy-1-4C-alkyl, halogen, 2-4C-alkenyl, 2-4C-alkynyl, fluoro-1-4C-alkyl or cyanomethyl,
- R3: is halogen, hydroxy-1-4C-alkyl, 1-4C-alkoxy-1-4C-alkyl, 1-4C-alkoxy-1-4C-alkoxy-1-4C-alkyl, 1-4C-alkoxycarbonyl, fluoro-1-4C-alkoxy-1-4C-alkyl or the radical -CO-NR31R32,
where
R31 is hydrogen, 1-7C-alkyl, hydroxy-1-4C-alkyl or 1-4C-alkoxy-1-4C-alkyl and
R32 is hydrogen, 1-7C-alkyl, hydroxy-1-4C-alkyl or 1-4C-alkoxy-1-4C-alkyl,
or where
R31 and R32 together and including the nitrogen atom to which they are attached form a pyrrolidino, piperidino or morpholino radical
- Arom: is a R4-, R5-, R6- and R7-substituted mono- or bicyclic aromatic radical selected from the group consisting of phenyl, naphthyl, pyrrolyl, pyrazolyl, imidazolyl, 1,2,3-triazolyl, indolyl, benzimidazolyl, furanyl (furyl), benzofuranyl (benzofuryl), thiophenyl (thienyl), benzothiophenyl (benzothienyl), thiazolyl, isoxazolyl, pyridinyl, pyrimidinyl, quinolinyl and isoquinolinyl,
where
R4 is hydrogen, 1-4C-alkyl, hydroxy-1-4C-alkyl, 1-4C-alkoxy, 2-4C-alkenyloxy, carboxyl, 1-4C-alkoxycarbonyl, carboxy-1-4C-alkyl, halogen, hydroxyl, aryl, aryl-1-4C-alkyl, aryloxy, aryl-1-4C-alkoxy, trifluoromethyl, nitro, amino, mono- or di-1-4C-alkylamino or sulfonyl,
R5 is hydrogen, 1-4C-alkyl, 1-4C-alkoxy, 1-4C-alkoxycarbonyl, halogen, trifluoromethyl or hydroxyl,
R6 is hydrogen, 1-4C-alkyl or halogen and
R7 is hydrogen, 1-4C-alkyl or halogen, where
aryl is phenyl or substituted phenyl having one, two or three identical or different substituents from the group consisting of 1-4C-alkyl, 1-4C-alkoxy, carboxyl, halogen, trifluoromethyl, nitro, trifluoromethoxy, hydroxyl and cyano,
and their salts.

Compounds which are to be mentioned are those compounds of the formula 1,
where
- R1: is 1-4C-alkyl or 3-7C-cycloalkyl
- R2: is hydrogen, 1-4C-alkyl, halogen or fluoro-1-4C-alkyl
- R3: is halogen, hydroxy-1-4C-alkyl, 1-4C-alkoxy-1-4C-alkyl, 1-4C-alkoxy-1-4C-alkoxy-1-4C-alkyl, 1-4C-alkoxycarbonyl, or the radical -CO-NR31 R32,
where
R31 is hydrogen, 1-7C-alkyl, hydroxy-1-4C-alkyl, 3-7C-cycloalkyl or 1-4C-alkoxy-1-4C-alkyl and
R32 is hydrogen, 1-7C-alkyl, hydroxy-1-4C-alkyl, 3-7C-cycloalkyl or 1-4C-alkoxy-1-4C-alkyl,
or where
R31 and R32 together and including the nitrogen atom to which they are attached form a pyrrolidino, piperidino or morpholino radical
- Arom: is a R4-, R5-, R6- and R7-substituted phenyl,
where
R4 is hydrogen or 1-4C-alkyl,
R5 is hydrogen or 1-4C-alkyl,
R6 is hydrogen or 1-4C-alkyl and
R7 is hydrogen,
and their salts.

Compounds which are also to be mentioned are those compounds of the formula 1,
where
- R1: is 1-4C-alkyl or 3-7C-cycloalkyl
- R2: is hydrogen, 1-4C-alkyl, halogen or fluoro-1-4C-alkyl
- R3: is halogen, hydroxy-1-4C-alkyl, 1-4C-alkoxy-1-4C-alkyl, 1-4C-alkoxy-1-4C-alkoxy-1-4C-alkyl, 1-4C-alkoxycarbonyl, or the radical -CO-NR31 R32,
where
R31 is hydrogen, 1-7C-alkyl, hydroxy-1-4C-alkyl or 1-4C-alkoxy-1-4C-alkyl and
R32 is hydrogen, 1-7C-alkyl, hydroxy-1-4C-alkyl or 1-4C-alkoxy-1-4C-alkyl,
or where
R31 and R32 together and including the nitrogen atom to which they are attached form a pyrrolidino, piperidino or morpholino radical
- Arom: is a R4-, R5-, R6- and R7-substituted phenyl,
where
R4 is hydrogen or 1-4C-alkyl,
R5 is hydrogen or 1-4C-alkyl,
R6 is hydrogen or 1-4C-alkyl and
R7 is hydrogen,
and their salts.

Compounds which are to be particularly mentioned are those of the formula 1,
where
- R1: is 1-4C-alkyl,
- R2: is 1-4C-alkyl,
- R3: is halogen, hydroxy-1-4C-alkyl, 1-4C-alkoxycarbonyl, or the radical -CO-NR31R32,
where
R31 is hydrogen, 1-7C-alkyl, hydroxy-1-4C-alkyl, 3-7C-cycloalkyl or 1-4C-alkoxy-1-4C-alkyl and
R32 is hydrogen, 1-7C-alkyl, hydroxy-1-4C-alkyl, 3-7C-cycloalkyl or 1-4C-alkoxy-1-4C-alkyl,
or where
R31 and R32 together and including the nitrogen atom to which they are attached form a pyrrolidino, piperidino or morpholino radical
- Arom: is phenyl,
and their salts.

Compounds which are also to be particularly mentioned are those of the formula 1,
where
- R1: is 1-4C-alkyl,
- R2: is 1-4C-alkyl,
- R3: is halogen, hydroxy-1-4C-alkyl, 1-4C-alkoxycarbonyl, or the radical -CO-NR31 R32,
where
R31 is hydrogen, 1-7C-alkyl, hydroxy-1-4C-alkyl or 1-4C-alkoxy-1-4C-alkyl and
R32 is hydrogen, 1-7C-alkyl, hydroxy-1-4C-alkyl or 1-4C-alkoxy-1-4C-alkyl,
or where
R31 and R32 together and including the nitrogen atom to which they are attached form a pyrrolidino, piperidino or morpholino radical
- Arom: is phenyl,
and their salts.

Compounds which are also to be particularly mentioned are those of the formula 1,
where
- R1: is 1-4C-alkyl,
- R2: is 1-4C-alkyl,
- R3: is halogen, 1-4C-alkoxycarbonyl, or the radical -CO-NR31 R32,
where
R31 is hydrogen, 1-4C-alkyl, hydroxy-1-4C-alkyl, 3-7C-cycloalkyl or 1-4C-alkoxy-1-4C-alkyl and
R32 is hydrogen, 1-4C-alkyl, hydroxy-1-4C-alkyl, 3-7C-cycloalkyl or 1-4C-alkoxy-1-4C-alkyl,
or where
R31 and R32 together and including the nitrogen atom to which they are attached form a pyrrolidino, piperidino or morpholino radical
- Arom: is phenyl,
and their salts.

Compounds which are also to be particularly mentioned are those of the formula 1,
where
- R1: is 1-4C-alkyl,
- R2: is 1-4C-alkyl,
- R3: is halogen, 1-4C-alkoxycarbonyl, or the radical -CO-NR31 R32,
where
R31 is hydrogen, 1-4C-alkyl, hydroxy-1-4C-alkyl or 1-4C-alkoxy-1-4C-alkyl and
R32 is hydrogen, 1-4C-alkyl, hydroxy-1-4C-alkyl or 1-4C-alkoxy-1-4C-alkyl,
or where
R31 and R32 together and including the nitrogen atom to which they are attached form a pyrrolidino, piperidino or morpholino radical
- Arom: is phenyl,
and their salts.

Compounds which are to be emphasized are those of the formula 1,
where
- R1: is 1-4C-alkyl,
- R2: is 1-4C-alkyl,
- R3: is halogen, 1-4C-alkoxycarbonyl, or the radical -CO-NR31 R32,
where
R31 is hydrogen, 1-4C-alkyl, hydroxy-1-4C-alkyl, 3-7C-cycloalkyl or 1-4C-alkoxy-1-4C-alkyl and
R32 is hydrogen, 1-4C-alkyl, hydroxy-1-4C-alkyl, 3-7C-cycloalkyl or 1-4C-alkoxy-1-4C-alkyl,
or where
R31 and R32 together and including the nitrogen atom to which they are attached form a pyrrolidino radical
- Arom: is phenyl,
and their salts.

Compounds which are also to be emphasized are those of the formula 1,
where
- R1: is 1-4C-alkyl,
- R2: is 1-4C-alkyl,
- R3: is halogen, 1-4C-alkoxycarbonyl, or the radical -CO-NR31 R32,
where
R31 is hydrogen, 1-4C-alkyl, hydroxy-1-4C-alkyl or 1-4C-alkoxy-1-4C-alkyl and
R32 is hydrogen, 1-4C-alkyl, hydroxy-1-4C-alkyl or 1-4C-alkoxy-1-4C-alkyl,
or where
R31 and R32 together and including the nitrogen atom to which they are attached form a pyrrolidino radical
- Arom: is phenyl,
and their salts.

Compounds which are also to be emphasized are those of the formula 1,
where
- R1: is 1-4C-alkyl,
- R2: is 1-4C-alkyl,
- R3: is halogen or 1-4C-alkoxycarbonyl,
- Arom: is phenyl,
and their salts

The invention further relates to the use of compounds of the formula 1, in which R1, R2, R3 and Arom have the meaning as indicated in the outset, for the preparation of compounds of the formula 2 and their salts 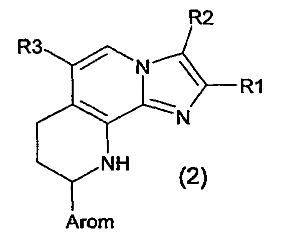 in which R1, R2, R3 and Arom have the meaning as indicated in the outset, which compounds are said to be suitable for treating gastrointestinal disorders.

The following exemplary compounds of the formula 2 (Table 1) can be synthesized from compounds of the formula 1 according to the general procedures outlined in more detail below. Further compounds of the formula 2 which are not listed in table 1 can likewise be prepared from the corresponding compounds of the formula 1.

**Table 1:**

| **R1** | **R2** | **R3** | **Arom** |
|---|---|---|---|
| | | | |
| CH₃ | CH₃ | CH₃OCH₂CH₂N(H)C(O)- | Phenyl |
| CH₃ | CH₃ | CH₃CH₂OC(O)- | Phenyl |
| CH₃ | CH₃ | CH₃N(H)C(O)- | Phenyl |
| CH₃ | CH₃ | HOCH₂CH₂N(H)C(O)- | Phenyl |
| CH₃ | CH₃ | (CH₃)₂N-C(O)- | Phenyl |
| CH₃ | CH₃ | H₂N-C(O)- | Phenyl |
| CH₃ | CH₃ | Morpholino-C(O)- | Phenyl |
| CH₃ | CH₃ | Pyrrolidino-C(O)- | Phenyl |
| CH₃ | CH₃ | HO-CH₂- | Phenyl |
| CH₃ | CH₃ | cyclopropyl-N(H)-C(O)- | Phenyl |

The invention thus further relates to the compounds of the formula 2 listed in the table 1 above and their salts.

Suitable salts of compounds of the formula 1 and of the formula 2 are - depending on the substitution - in particular all acid addition salts. Particular mention may be made of the pharmacologically acceptable salts of the inorganic and organic acids customarily used in pharmacy. Those suitable are watersoluble and water-insoluble acid addition salts with acids such as, for example, hydrochloric acid, hydrobromic acid, phosphoric acid, nitric acid, sulfuric acid, acetic acid, citric acid, D-gluconic acid, benzoic acid, 2-(4-hydroxybenzoyl)benzoic acid, butyric acid, sulfosalicylic acid, maleic acid, lauric acid, malic acid, fumaric acid, succinic acid, oxalic acid, tartaric acid, embonic acid, stearic acid, toluenesulfonic acid, methanesulfonic acid or 3-hydroxy-2-naphthoic acid, where the acids are employed in the salt preparation in an equimolar ratio or in a ratio differing therefrom, depending on whether the acid is a mono- or polybasic acid and on which salt is desired.

Pharmacologically unacceptable salts, which can be initially obtained, for example, as process products in the preparation of the compounds of the formula 1 or of the formula 2 according to the invention on an industrial scale, are converted into pharmacologically acceptable salts by processes known to the person skilled in the art.

It is known to the person skilled in the art that the compounds of the formula 1 or of the formula 2 according to the invention and their salts can, for example when they are isolated in crystalline form, comprise varying amounts of solvents. The invention therefore also embraces all solvates and, in particular, all hydrates of the compounds of the formula 1 or of the formula 2, and all solvates and, in particular, all hydrates of the salts of the compounds of the formula 1 or of the formula 2.

The preparation of the compounds of the formula 2 is performed, as shown for example in scheme 1, such that the compounds of the formula 1, in which R1, R2, R3 and Arom have the meanings as indicated in the outset, are subjected to a cyclization reaction, for example under acidic conditions, using for example sulphuric acid.

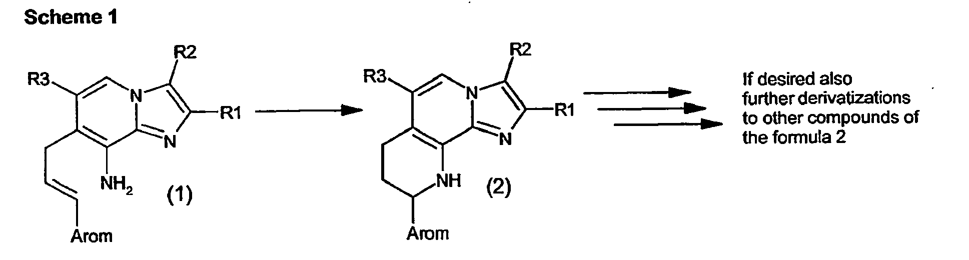

Compounds of the formula 2 obtained according to Scheme 1 can be subjected, if desired, to further derivatizations. If compounds of the formula 2 with, for example, R3 = Br are obtained, further chemical transformations, which are known per se, can be performed to synthesize a great variety of other compounds of the formula 2 by reactions known to the expert. If, for example, compounds where R3 = -CO-1-4C-alkoxy or R3 = -CO-NR31 R32 are desired, an appropriate derivatization can be performed in a manner known per se (e. g. metal catalysed carbonylation of the corresponding bromo compound or conversion of an ester into an amide).

The compounds of the formula 1 according to the invention, in which R1, R2, R3 and Arom have the meanings as indicated in the outset, can be prepared, for example, following the reaction sequence as outlined in Scheme 2 under standard reaction conditions, as described for example in more detail in the examples.

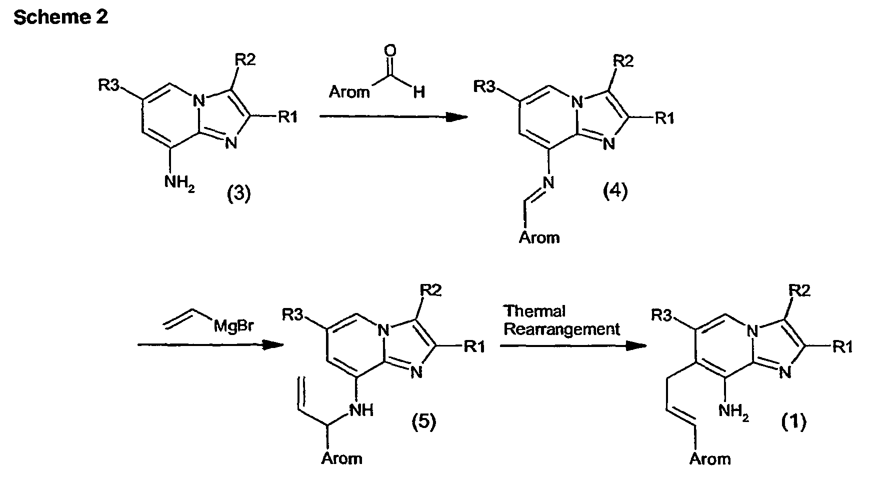

Compounds of the formula 3 with R3 = 1-4C-alkoxycarbonyl or -CONR31 R32 are known for example from WO 02/20523 or WO 99/55706 or can be prepared with the same or other substituents R3 (for example R3 = halogen) in a manner known to the expert, for example in analogy to the synthesis disclosed in J. Med. Chem. 1985, 28, 876-892.

Compounds of the formula 1 obtained according to Scheme 2 can be subjected, if desired, to further derivatization reactions. If compounds of the formula 1 with, for example, R3 = 1-4C-alkoxycarbonyl are obtained, further chemical transformations, which are known per se, can be performed to synthesize a great variety of other compounds of the formula 1 by reactions known to the expert. If, for example, compounds of the formula 1 where R3 = -CO-NR31 R32 are desired, an appropriate derivatization can be performed by methods known to the expert (e.g. conversion of an ester into a carboxylic acid and further into an amide), whereby protection of the amino functionality in 8-position with a suitable protecting group, for example in form of a bis-*tert*-butyloxycarbonyl-amino group, may be required during the derivatization reactions.

Alternatively, for example compounds of the formula (1) where R3 = -CO-NR31R32 can be prepared according to schema 3 by derivatization of the substituent R3 on the stage of compounds of the formula 5. If compounds of the formula 1 with, for example, R3 = 1-4C-alkoxycarbonyl are obtained, further chemical transformations, which are known per se, can be performed to synthesize a great variety of other compounds of the formula 1 by reactions known to the expert.

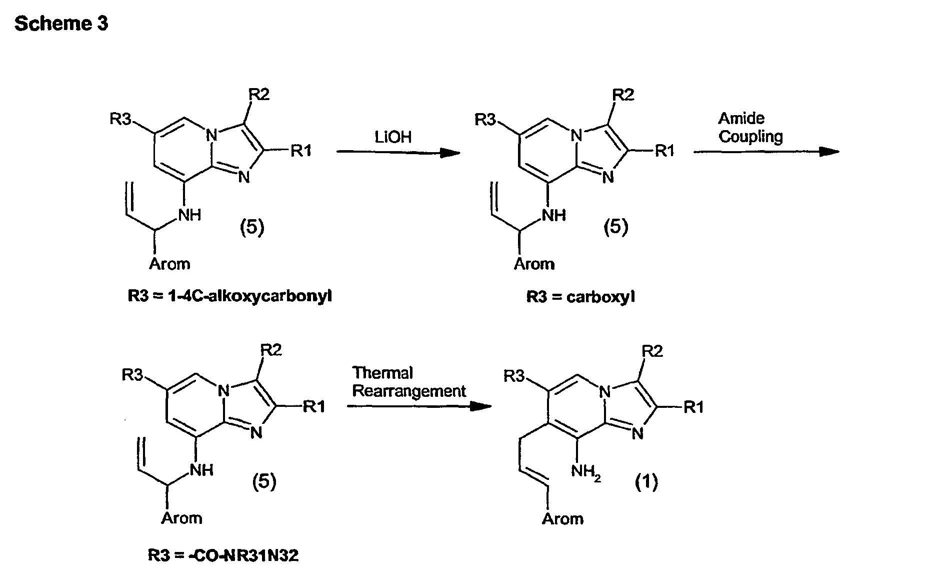

The compounds of the formula 2 have at least one center of chirality in the skeleton. The invention thus provides all feasible enantiomers of compounds of the formula 2 in any mixing ratio, including the pure enantiomers, which are a preferred subject matter of the invention.

The compounds of the formula 2a, which are a preferred subject matter of the invention, 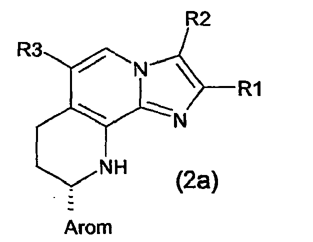 can be isolated from the corresponding racemic mixtures of the formula 2 by separating the compounds of the formula 2a from their optical antipodes by techniques known to the expert. The separation can be achieved for example by crystallization of diastereomeric salts after reaction of the racemic mixture of acid free compounds of the formula 2 with a suitable, optically pure acid or by preparative chromatography using a chiral column, as described in an exemplary manner in the examples, or by other methods known to the expert.

Among the compounds of the formula 2a, those compounds are preferred wherein R1, R2, R3 and Arom have the meanings as described above for the compounds of the formula 1.

Although enantiomerically pure tricyclic imidazo[1,2-a]pyridine derivatives are known for example from the International Patent Application WO 95/27714, the higher activity of the compounds of the formula 2a as compared to their optical antipodes was unexpected. So far, the preference for enantiomers of the formula 2a due to a more pronounced activity in inhibiting gastric acid secretion as compared to their optical antipodes has not been described yet for any combination of the substituents R1, R2, R3 and Arom.

It has further been found, unexpectedly, that the enantiomers of the formula 2a have a pronounced activity in inhibiting gastric acid secrection as compared to their optical antipodes.

Preferred exemplary compounds of the formula 2a are those in which R1, R2, R3 and Arom have the meanings as listed in table Table 2.

**Table 2:**

| **R1** | **R2** | **R3** | **Arom** |
|---|---|---|---|
| CH₃ | CH₃ | CH₃OCH₂CH₂N(H)C(O)- | Phenyl |
| CH₃ | CH₃ | CH₃CH₂O(O)- | Phenyl |
| CH₃ | CH₃ | CH₃N(H)C(O)- | Phenyl |
| CH₃ | CH₃ | HOCH₂CH₂N(H)C(O)- | Phenyl |
| CH₃ | CH₃ | (CH₃)₂N-C(O)- | Phenyl |
| CH₃ | CH₃ | H₂N-C(O)- | Phenyl |
| CH₃ | CH₃ | Morpholino-C(O)- | Phenyl |
| CH₃ | CH₃ | Pyrrolidino-C(O)- | Phenyl |
| CH₃ | CH₃ | HO-CH₂- | Phenyl |
| CH₃ | CH₃ | cydopropyl-N(H)-C(O)- | Phenyl |

The invention thus further relates to the compounds of the formula 2a in which R1, R2, R3 and Arom have the meanings as indicated in the outset for the compounds of the formula 1.

The invention further relates to those compounds of the formula 2a listed in table 2 above, and their salts.

The following examples serve to illustrate the invention in greater detail without restricting it. Likewise, further compounds of the formula 1, formula 2 and formula 2a, whose preparation is not described explicitly can be prepared in an analogous manner or in a manner familiar per se to the person skilled in the art using customary process techniques. The abbreviation min stands for minute(s), h for hour(s) and m.p. for melting point.

### Examples

### I. Compounds of the formula 1

### 1. 6-Bromo-2,3-dimethyl-7-((E)-3-phenyl-allyl)-imidazo[1,2-a]pyridin-8-ylamine

40 g (105 mmol) (6-bromo-2,3-dimethyl-imidazo[1,2-*a*]pyridin-8-yl)-(1-phenyl-allyl)-amine were melted at 220° C. After 4h, the reaction was cooled to room temperature and the residue was crystallized from diisopropyl ether to afford 20.8 g (56%) of the title compound as a beige solid. m.p. 128° C.

### 2. 8-Amino-2,3-dimethyl-7-((E)-3-phenyl-allyl)-imidazo[1,2-a]pyridine-6-carboxylic Acid Methyl Ester

18 g (53.7 mmol) 2,3-dimethyl-8-(1-phenyl-allylamino)-imidazo[1,2-a]pyridine-6-carboxylic acid methyl ester in 80 ml N,N-dimethylaniline were heated at 220° C for 22h. After cooling to room temperature, the reaction was concentrated in vacuo and the residue crystallized from diisopropyl ether to afford 9.4 g (52%) of the title compound as a brown solid. m.p. 118°-120° C.

### 3. 8-Amino-2,3-dimethyl-7-((E)-3-phenylallyl)-imidazo[1,2-a]pyridine-6-carboxylic Acid Methylamide

A solution of 0.23 g (0.43 mmol) 8-(bis-*tert*-butyloxycarbonylamino)-2,3-dimethyl-7-((E)-3-phenyl-allyl)-imidazo[1,2-a]pyridine-6-carboxylic acid methylamide in 10 ml dichloromethane was treated with 0.025 g (0.22 mmol) trifluoroacetic acid and the reaction mixture was stirred for 16 h at room temperature. The solution was adjusted to pH = 6 by adding 6N sodium hydroxide solution and extracted with dichloromethane. The organic layers were dried over magnesium sulfate and concentrated in vacuo. The residue was purified by column chromatography on silica gel (dichloromethane/methanol = 9:1) and crystallized from acetone to afford 0.15 g (100%) of the title compound as a white solid. m.p. 204°-208° C.

### 4. 8-Amino-2,3-dimethyl-7-((E)-3-phenyl-allyl)-imidazo[1,2-a]pyridine-6-carboxylic Acid Dimethylamide

A solution of 0.4 g (0.54 mmol) 8-(bis-*tert*-butyloxycarbonylamino)-2,3-dimethyl-7-((E)-3-phenyl-allyl)-imidazo[1,2-a]pyridine-6-carboxylic acid dimethylamide in 10 ml dichloromethane was treated with 0.04 g (0.28 mmol) trifluoroacetic acid and the reaction mixture was stirred for 16 h at room temperature. The solution was poured into water, neutralized with saturated sodium bicarbonate solution and extracted with dichloromethane (3 x 20 ml). The organic layers were dried over magnesium sulfate and concentrated in vacuo. The residue was purified by column chromatography on silica gel (dichloromethane/methanol = 9:1) to afford 0.16 g (84%) of the title compound as a brown solid. m.p. 163°-167° C

### 5. 8-Amino-2,3-dimethyl-7-((E)-3-phenyl-allyl)-imidazo[1,2-a]pyridine-6-carboxylic Acid (2-hydroxy-ethyl)-amide

A solution of 0.2 g (0.53 mmol) 8-(bis-*tert*-butyloxycarbonylamino)-2,3-dimethyl-7-((E)-3-phenyl-allyl)-imidazo[1,2-a]pyridine-6-carboxylic acid (2-hydroxy-ethyl)-amide in 10 ml dichloromethane was treated with 0.03 g (0.27 mmol) trifluoroacetic acid and the reaction mixture was stirred for 16 h at room temperature. The solution was adjusted to pH = 6 by adding 6N sodium hydroxide solution and extracted with dichloromethane. The organic layers were dried over magnesium sulfate and concentrated in vacuo. The residue was purified by column chromatography on silica gel (dichloromethane/methanol = 9:1) and crystallized from diethyl ether to afford 0.15 g (75%) of the title compound as a yellow solid. m.p. 113°-115° C.

### 6. 1-[8-Amino-2,3-dimethyl-7-((E)-3-phenyl-allyl)-imidazo[1,2-a]pyridin-6-yl]-1-pyrrolidin-1-yl-methanone Hydrochloride

A solution of 0.5 g (0.89 mmol) 1-18-(bis-*tert*-butyloxycarbonylamino)-2,3-dimethyl-7-((E)-3-phenyl-allyl)-imidazo[1,2-a]pyridin-6-yl]-1-pyrrolidin-1-yl-methanone in 15 ml dichloromethane was treated with 0.05 g (0.4 mmol) trifluoroacetic acid and the reaction mixture was stirred for 16 h at room temperature. The solution was adjusted to pH = 6 by adding 6N sodium hydroxide solution and extracted with dichloromethane. The organic layers were dried over magnesium sulfate and concentrated in vacuo. The residue was purified by column chromatography on silica gel (dichloromethane/methanol = 9:1) and then treated with etheric HCl solution in acetone to afford 0.18 g (58%) of the title compound as a white sol id. m.p. 229°-231° C.

### 7. 8-Amino-2,3-dimethyl-7-((E)-3-phenyl-allyl)-imidazo[1,2-a]pyridine-6-carboxylic acid (2-methoxy-ethyl)-amide

A solution of 0.5 g (0.86 mmol) 8-(bis-*tert*-butyloxycarbonylamino)-2,3-dimethyl-7-((E)-3-phenyl-allyl)-imidazo[1,2-a]pyridine-6-carboxylic acid (2-methoxy-ethyl)-amide in 15 ml dichloromethane was treated with 0.05 g (0.43 mmol) trifluoroacetic acid and the reaction mixture was stirred for 16 h at room temperature. The solution was adjusted to pH = 6 by adding 6N sodium hydroxide solution and extracted with dichloromethane. The organic layers were dried over magnesium sulfate and concentrated in vacuo. The residue was purified by column chromatography on silica gel (dichloromethane/methanol = 9:1) and crystallized from diethyl ether-isopropanol to afford 0.22 g (70%) of the title compound as a yellow solid. m.p. 204°-208° C.

### 8. 8-Amino-2,3-dimethyl-7-((E)-3-phenyl-allyl)-imidazo[1,2-a]pyridine-6-carboxylic acid amide

A solution of 0.27 g (0.52 mmol) 8-(bis-*tert*-butyloxycarbonylamino)-2,3-dimethyl-7-((E)-3-phenyl-allyl)-imidazo[1,2-a]pyridine-6-carboxylic acid amide in 10 ml dichloromethane was treated with 0.03 g (0.28 mmol) trifluoroacetic acid and the reaction mixture was stirred for 16 h at room temperature. The solution was adjusted to pH = 6 by adding 6N sodium hydroxide solution and extracted with dichloromethane. The organic layers were dried over magnesium sulfate and concentrated in vacuo. The residue was purified by column chromatography on silica gel (dichloromethane/methanol = 9:1) and crystallized from diethyl ether to afford 0.16 g (100%) of the title compound as a brown solid. m.p. 83°-87° C.

### II. Compounds of the formula 2

### a. 2,3-Dimethyl-8-phenyl-6,7,8,9-tetrahydro-1,3a,9-triaza-cyclopenta[a]naphthalene-5-carboxylic acid-(2-methoxy-ethyl)-amide

To a suspension of 0.2 g (0.64 mmol) 2,3-dimethyl-8-phenyl-6,7,8,9-tetrahydro-1,3a,9-triaza-cyclopenta[a]-naphthalene-5-carboxylic acid in dichloromethane (12 ml) were added 0.2 g (0.64 mmol) O-(1H-benzotriazol-1-yl)-N,N,N', N'-tetramethyluronium tetrafluoroborate (TBTU) and the reaction was stirred at room temperature for 30 min.. 278 µl (3.2 mmol) 2-Methoxy-ethylamine were added and the reaction mixture was stirred at room temperature for 4 h. The solution was poured into water (15 ml) and extracted with dichloromethane (4 x 20 ml). The organic layers were dried over magnesium sulphate and concentrated in vacuo. The residue was purified by column chromatography on silica gel (dichloromethane/ methanol = 15:1) to afford 0.1 g (44%) of the title compound as a yellow solid. m.p. 178°-180° C.

### b. 2,3-Dimethyl-8-phenyl-6,7,8,9-tetrahydro-1,3a,9-triaza-cyclopenta[a]naphthalene-5-carboxylic Acid Ethyl Ester

To a solution of 6.8 g (19.1 mmol) 5-bromo-2,3-dimethyl-8-phenyl-6,7,8,9-tetrahydro-1,3a, 9-triaza-cyclo-penta[*a*]naphthalene in 300 ml ethanol were added 2.0 g (7.6 mmol) triphenylphosphine, 16 ml (124.1 mmol) triethylamine and 0.64 g (2.9 mmol) palladium(II) acetate. The mixture was transferred to an autoclave and carbonylated (6 bar carbon monoxide pressure, 100° C) for 20h. The catalyst was filtered off and the filtrate concentrated in vacuo. The residue was purified by chromatography on silica gel (ethyl acetate/ petroleum ether = 2:1) to afford 6.1 g (91%) of the title compound as a yellow solid. m.p. 96°-101°C.

### c. 2,3-Dimethyl-8-phenyl-6,7,8,9-tetrahydro-1,3a,9-triaza-cyclopenta[a]naphthalene-5-carboxylic Acid Methylamide

To a suspension of 1.0 g (3.1 mmol) 2,3-dimethyl-8-phenyl-6,7,8,9-tetrahydro-1,3a,9-triaza-cyclopenta[a]-naphthalene-5-carboxylic acid in dichloromethane (30 ml) were added 1.1 g (3.4 mmol) O-(1H-benzotnazol-1-yl)-N,N,N', N'-tetramethyluronium tetrafluoroborate (TBTU) and the reaction was stirred at room temperature for 2h. 6.2 ml (12.4 mmol) Methylamine (2M in THF) were added and the reaction mixture was stirred at room temperature for 42 h. The solution was poured into water (30 ml) and extracted with dichloromethane (3 x 15 ml). The organic layers were dried over magnesium sulphate and concentrated in vacuo. Purification of the residue by column chromatography on silica gel (dichloromethane/ methanol = 10:1) and crystallization from diisopropyl ether/ ethyl acetate (3:1) afforded 0.3 g (31%) of the title compound as a yellow solid. m.p. 245°-248° C.

### d. 2,3-Dimethyl-8-phenyl-6,7,8,9-tetrahydro-1,3a,9-triaza-cyclopenta[a]naphthalene-5-carboxylic Acid (2-Hydroxy-ethyl)-amide

To a suspension of 1.0 g (3.1 mmol) 2,3-dimethyl-8-phenyl-6,7,8,9-tetrahydro-1,3a,9-triaza-cyclopenta[a]-naphthalene-5-carboxylic acid in dichloromethane (50 ml) were added 1.5 g (4.7 mmol) O-(1H-benzotriazol-1-yl)-N,N,N', N'-tetramethyluronium tetrafluoroborate (TBTU) and the reaction was stirred at room temperature for 2h. 943 µl (4.7 mmol) Ethanolamine were added and the reaction was stirred overnight at room temperature. The solution was poured into water (50 ml) and extracted with dichloromethane (3 x 30 ml). The organic layers were dried over magnesium sulphate and concentrated in vacuo. The residue was crystallized from ethyl acetate/diisopropyl ether (5:1) to afford 0.8 g (69%) of the title compound as a yellow solid. m.p. 242°-244° C.

### e. 2,3-Dimethyl-8-phenyl-6,7,8,9-tetrahydro-1,3a,9-triaza-cyclopenta[a]naphthalene-5-carboxylic Acid Dimethylamide

To a suspension of 0.6 g (1.9 mmol) 2,3-dimethyl-8-phenyl-6,7,8,9-tetrahydro-1,3a,9-triaza-cyclopenta[a]-naphthalene-5-carboxylic acid in dichloromethane (30 ml) were added 0.6 g (2.1 mmol) O-(1H-benzotriazol-1-yl)-N,N,N', N'-tetramethyluronium tetrafluoroborate (TBTU) and the suspension was stirred at room temperature for 2h. 3.7 ml (7.5 mmol) Dimethylamine (2M in THF) were added and the reaction mixture was stirred overnight at room temperature. The solution was poured into water (30 ml) and extracted with dichloromethane (2 x 20 ml). The organic layers were dried over magnesium sulphate and concentrated in vacuo. The residue was purified by column chromatography on silica gel (dichloromethane/ methanol = 14:1) to afford 0.5 g (74%) of the title compound as a beige solid. m.p. 185°-187° C.

### f. 2,3-Dimethyl-8-phenyl-6,7,8,9-tetrahydro-1,3a,9-triaza-cyclopenta[a]naphthalene-5-carboxylic Acid Amide

To a suspension of 0.6 g (1.9 mmol) 2,3-dimethyl-8-phenyl-6,7,8,9-tetrahydro-1,3a,9-triaza-cyclopenta[a]-naphthalene-5-carboxylic acid in dichloromethane (30 ml) were added 0.6 g (2.1 mmol) O-(1H-benzotriazol-1-yl)-N,N,N', N'-tetramethyluronium tetrafluoroborate (TBTU) and the suspension was stirred at room temperature for 2h. 22.8 ml (11.4 mmol) Ammonia (0.5 M in dioxane) were added and the solution was stirred overnight at room temperature. The reaction mixture was poured into water (30 ml) and extracted with dichloromethane (3 x 15 ml). The organic layers were dried over magnesium sulphate and concentrated in vacuo. The residue was purified by column chromatography on silica gel (dichloromethane/ methanol = 10:1) to afford 0.33 g (55%) of the title compound as a beige solid. m.p. 331°-332° C.

### g. 1-(2,3-Dimethyl-8-phenyl-6,7,8,9-tetrahydro-1,3a,9-triaza-cyclopenta[a]naphthalen-5-yl)-1-morpholin-4-yl-methanone

To a suspension of 0.3 g (0.9 mmol) 2,3-dimethyl-8-phenyl-6,7,8,9-tetrahydro-1,3a,9-triaza-cyclopenta[a]-naphthalene-5-carboxylic acid in dichloromethane (10 ml) were added 0.3 g (1.0 mmol) O-(1H-benzotriazol-1-yl)-N,N,N', N'-tetramethyluronium tetrafluoroborate (TBTU) and the suspension was stirred overnight at room temperature. 431 µl (4.9 mmol) Morpholine were added and the solution was stirred at room temperature for 5h. The reaction mixture was poured into water (30 ml) and extracted with dichloromethane (3 x 20 ml). The organic layers were dried over magnesium sulphate and concentrated in vacuo. The residue was purified by column chromatography on silica gel (dichloromethane/ methanol = 20: 1) and crystallized from diethyl ether to afford 0.18 g (50%) of the title compound as a white solid. m.p. 155°-156° C.

### h. 1-(2,3-Dimethyl-8-phenyl-6,7,8,9-tetrahydro-1,3a,9-triaza-cyclopenta[a]naphthalen-5-yl)-1-pyrrolidin-1-yl-methanone

To a suspension of 0.32 g (1.0 mmol) 2,3-dimethyl-8-phenyl-6,7,8,9-tetrahydro-1,3a,9-triaza-cyclopenta[a]-naphthalene-5-carboxylic acid in dichloromethane (10 ml) were added 0.34 g (1.1 mmol) O-(1H-benzotriazol-1-yl)-N,N,N', N'-tetramethyluronium tetrafluoroborate (TBTU) and the suspension was stirred overnight at room temperature. 413 µl (4.9 mmol) Pyrrolidine were added and the solution was stirred at room temperature for 5h. The reaction mixture was poured into water (30 ml) and extracted with dichloromethane (3 x 20 ml). The organic layers were dried over magnesium sulphate and concentrated in vacuo. The residue was purified by column chromatography on silica gel (dichloromethane/ methanol = 20: 1) and crystallized from diethyl ether to afford 0.12 g (32%) of the title compound as a yellow solid. m.p. 201 °-203° C.

### i. (2,3-Dimethyl-8-phenyl-6,7,8,9-tetrahydro-1,3a,9-triaza-cyclopenta[a]naphthalen-5-yl)-methanol

413 mg (1.2 mmol) 2,3-Dimethyl-8-phenyl-6,7,8,9-tetrahydro-1,3a,9-triaza-cyclopenta[a]naphthalene-5-carboxylic acid ethyl ester were dissolved in 8 ml tetrahydrofurane and the solution was cooled to 0° C. The reaction mixture was stirred for 2 h, poured into water (5 ml) and extracted with dichloromethane (4 x 50 ml). The organic layer was dried over magnesium sulphate and concentrated in vacuo. The residue was purified by column chromatography on silica gel (dichloromethane/methanol = 25:1) to afford 220 mg (61 %) of the title compound as a yellow solid. m.p. 226°-230° C.

### j. (8S)-2,3-Dimethyl-8-phenyl-6,7,8,9-tetrahydro-1,3a,9-triaza-cyclopenta[a]naphthalene-5-carboxylic Acid Dimethylamide

Resolution of racemic 2,3-dimethyl-8-phenyl-6,7,8,9-tetrahydro-1,3a,9-triaza-cyclopenta[a]-naphthalene-5-carboxylic acid dimethylamide (1.7 g, 4.9 mmol)) was achieved by preparative chromatography using a 250 x 50 mm CHIRALPAK® AD 20 µm column. The mobile phase consisted of a n-heptan / isopropanol mixture [85/15 (v/v)]. The separation was performed at room temperature with a flow rate of 120 ml/min. The products were detected at a wavelength of 280 nm. The separation afforded 0.73 g (50%; ee 100%) of the title compound ((8*S*)-enantiomer) as a yellow solid. m.p. 185° C.

The optical purity was examined by means of optical rotation. For the title compound ((8S)-enantiomer) an [α]^{D}₂₀ value of -72° (c = 0.1, dichloromethane) was determined.

### k. (8R)-2,3-Dimethyl-8-phenyl-6,7,8,9-tetrahydro-1,3a,9-triaza-cyclopenta[a]naphthalene-5-carboxylic Acid Dimethylamide

The separation of the title compound ((8*R*)-enantiomer) was performed as described in example j and afforded 0.75 g (50%; ee 99.7%) of the title compound as a yellow solid. m.p. 183° C. The [α]^{D}₂₀ value measured for the title compound ((8*R*)-enantiomer) was +72 ° (*c* = 0.1, dichloromethane).

### l. (8R)-2,3-Dimethyl-8-phenyl-6,7,8,9-tetrahydro-1,3a,9-triaza-cyclopenta[a]naphthalene-5-carboxylic Acid Methylamide

Resolution of racemic 2,3-dimethyl-8-phenyl-6,7,8,9-tetrahydro-1,3a,9-triaza-cyclopenta[a]-naphthalene-5-carboxylic acid methylamide (426 mg, 1.3 mmol)) was achieved by preparative chromatography using a 250 x 20 mm CHIRALCEL^{®} OD-H 5 µm column. The mobile phase consisted of a acetonitrile / diethylamine mixture [100/0.1 (v/v)]. The separation was performed at room temperature with a flow rate of 20 ml/min. The products were detected at a wavelength of 350 nm. The separation afforded 0.20 g (50%; ee > 99.5%) of the title compound ((8*R*)-enantiomer) as a yellow solid. m.p. 263-264° C.

The optical purity was examined by means of optical rotation. For the title compound ((8R)-enantiomer) an [α]^{D}₂₀ value of +82° (*c*= 1.16, chloroform) was determined.

### m. (8S)-2,3-Dimethyl-8-phenyl-6,7,8,9-tetrahydro-1,3a,9-triaza-cyclopenta[a]naphthalene-5-carboxylic Acid Methylamide

The separation of the title compound ((8*S*)-enantiomer) was performed as described in example I and afforded 0.20 g (50%; ee > 99.5%) of the title compound as a yellow solid. m.p. 263-264° C.
The [α]^{D}₂₀ value measured for the tittle compound ((8*S*)-enantiomer) was - 94 ° (*c* = 1.16, chloroform).

### n. (8R)-2,3-Dimethyl-8-phenyl-6,7,8,9-tetrahydro-1,3a,9-triaza-cyclopenta[a]naphthalene-5-carboxylic Acid Cyclopropylamide

Resolution of racemic 2,3-dimethyl-8-phenyl-6,7,8,9-tetrahydro-1,3a,9-triaza-cyclopenta[a]-naphthalene-5-carboxylic acid cyclopropylamide (163 mg, 0.4 mmol)) was achieved by preparative chromatography using a 250 x 20 mm CHIRALCEL® OD-H 5 µm column. The mobile phase consisted of a acetonitrile / diethylamine mixture [100/0.1 (v/v)]. The separation was performed at room temperature with a flow rate of 20 ml/min. The products were detected at a wavelength of 350 nm. The separation afforded 45 mg (31%; ee > 99.5%) of the tittle compound ((8*R*)-enantiomer) as a yellow solid. m.p. 249-251° C.

The optical purity was examined by means of optical rotation. For the title compound ((8R)-enantiomer) an [α]^{D}₂₀ value of + 55° (*c* = 1.22, chloroform) was determined.

### o. (8S)-2,3-Dimethyl-8-phenyl-6,7,8,9-tetrahydro-1,3a,9-triaza-cyclopenta[a]naphthalene-5-carboxylic Acid Cyclopropylamide

The separation of the title compound ((8*S*)-enantiomer) was performed as described in example n and afforded 69 mg (48%; ee > 99.5%) of the title compound as a yellow solid. m.p. 252-253° C.
The [α]^{D}₂₀ value measured for the title compound ((8*S*)-enantiomer) was - 71 ° (*c* = 0.98, chloroform).

### p. (8R)-2,3-Dimethyl-8-phenyl-6,7,8,9-tetrahydro-1,3a,9-triaza-cyclopenta[a]naphthalene-5-carboxylic Acid Amide

Resolution of racemic 2,3-dimethyl-8-phenyl-6,7,8,9-tetrahydro-1,3a,9-triaza-cyclopenta[a]-naphthalene-5-carboxylic acid amide (80 mg, 0.25 mmol)) was achieved by preparative chromatography using a 250 x 20 mm CHIRALCEL^{®} OD-H 5 µm column. The mobile phase consisted of a acetonitrile / ethanol / diethylamine mixture [95/5/0.1 (v/vlv)]. The separation was performed at room temperature with a flow rate of 20 ml/min. The products were detected at a wavelength of 340 nm. The separation afforded 35 mg (44%; ee 97%) of the title compound ((8*R*)-enantiomer) as a yellow solid. m.p. > 300° C.

The optical purity was examined by means of optical rotation. For the title compound ((8R)-enantiomer) an [α]^{D}₂₀ value of + 73° (*c* = 0.4, chloroform) was determined.

### q. (8S)-2,3-Dimethyl-8-phenyl-6,7,8,9-tetrahydro-1,3a,9-triaza-cyclopenta[a]naphthalene-5-carboxylic Acid Amide

The separation of the title compound ((8*S*)-enantiomer) was performed as described in example p and afforded 34 mg (44%; ee 90%) of the title compound as a yellow solid. m.p. > 300° C. The [α]^{D}₂₀ value measured for the title compound ((8*S*)-enantiomer) was - 133 ° (*c* = 0.4, chloroform).

### r. 2,3-Dimethyl-8-phenyl-6,7,8,9-tetrahydro-1,3a,9-triazarcyclopenta[a]naphthalene-5-carboxylic Acid Cyclopropylamide

To a suspension of 0.30 g (0.93 mmol) 2,3-dimethyl-8-phenyl-6,7,8,9-tetrahydro-1,3a,9-triaza-cyclopenta[a]-naphthalene-5-carboxylic acid in dichloromethane (10 ml) were added 0.33 g (1.0 mmol) O-(1H-benzotriazol-1-yl)-N,N,N', N'-tetramethyluronium tetrafluoroborate (TBTU) and the suspension was stirred overnight at room temperature. 65 µl (3.8 mmol) Cyclopropylamine were added and the solution was stirred at room temperature for 16h. The reaction mixture was poured into water (10 ml) and extracted with dichloromethane (3 x 10 ml). The organic layers were dried over magnesium sulphate and concentrated in vacuo. The residue was purified by column chromatography on silica gel (dichloromethane/ methanol = 20:1) to afford 0.2 g (60%) of the title compound as a ochre solid. m.p. 267°-269° C.

### III. Starting compounds and intermediates

### A. 6-Bromo-2,3-dimethyl-imidazo[1,2-a]pyridin-8-ylamine

To a solution of 50 g (266 mmol) 5-bromo-pyridine-2,3-diamine in 1I dioxane were added 56 ml (532 mmol) 3-bromo-butan-2-one and the reaction was refluxed for 6h. The precipitate was filtered and washed with methanol to give 52.3 g of the title compound as its hydrobromidric salt. The product was suspended in 500 ml water, neutralized with sodium bicarbonate and extracted with ethyl acetate (3 x 200 ml). The organic layers were dried over magnesium sulphate and concentrated in vacuo. The residue afforded 36.8 g (58%) of the title compound as a beige solid. m.p. 160°-163° C.

### B. Benzylidene-(6-bromo-2,3-dimethyl-imidazo[1,2-a]pyridin-8-yl)-amine

To a solution of 20.6 g (85.8 mmol) 6-bromo-2,3-dimethyl-imidazo[1,2-a]pyridin-8-ylamine in 1I toluene were added 13.1 ml (128.7 mmol) benzaldehyde and the reaction mixture was refluxed (Dean-Stark apparatus) for 60h under azeotropic removal of water. The solution was concentrated in vacuo and the residue was crystallized from diisopropyl ether to afford 20.8 g (74%) of the title compound as a yellow solid. m.p. 121°-126° C.

### C. (6-Bromo-2,3-dimethyl-imidazo[1,2-a]pyridin-8-yl)-(1-phenyl-allyl)-amine

A solution of 34.7 g (105.7 mmol) benzylidene-(6-bromo-2,3-dimethyl-imidazo[1,2-*a*]pyridin-8-yl)-amine in 1I dichloromethane was cooled to -60° C and 317 ml (317 mmol) vinylmagnesiumbromide (1M in THF) were added dropwise. After 2h, the solution was poured into 400 ml saturated aqueous ammonium chloride solution and extracted with dichloromethane (3 x 200 ml). The organic layers were dried over magnesium sulphate and concentrated in vacuo. The residue afforded 40 g (100%) of the title compound as a green oil.

### D. 5-Bromo-2,3-dimethyl-phenyl-6,7,8,9-tetrahydro-1,3a,9-triaza-cyclopenta[a]naphtalene

20.8 g (58.3 mmol) 6-Bromo-2,3-dimethyl-7-((E)-3-phenyl-allyl)-imidazo[1,2-a]pyridin-8-ylamine were suspended in 500 ml 70% sulfuric acid and the reaction was heated at 100° C for 2h. The solution was adjusted to pH = 7 with 10 M sodium hydroxide solution and extracted with ethyl acetate. The organic layer was dried over magnesium sulphate and concentrated in vacuo. The residue was purified by column chromatography on silica gel (ethyl acetate/ petroleum ether = 3:1) to afford 7.8 g (38%) of the title compound as a beige solid. m.p. 125°-129° C.

### E. 2,3-dimethyl-8-phenyl-6,7,8,9-tetrahydro-1,3a,9-triaza-cyclopenta[a]-naphthalene-5-carboxylic acid

To a solution of 6.0 g (17.2 mmol) 2,3-dimethyl-8-phenyl-6,7,8,9-tetrahydro-1,3a,9-triaza-cyclopenta[a] naphthalene-5-carboxylic acid ethyl ester in 290 ml tetrahydrofuran was added a solution of 2.5 g (103 mmol) lithium hydroxide in 85 ml water and the mixture was refluxed for 12h. The reaction was cooled to room temperature and concentrated in vacuo. The residue was dissolved in water (100 ml) and the solution adjusted to pH = 4-5 with 2M HCl. The precipitate was filtered off and dried in vacuo at 40° C to afford 4.8 g (87%) of the title compound as a yellow solid. m.p. 264°-268° C.

### F. 8-(Benzylidene-amino)-2,3-dimethyl-imidazo[1,2-a]pyridine-6-carboxylic acid methyl ester

To a suspension of 2.0 g (9.1 mmol) 8-amino-2,3-dimethyl-imidazo[1,2-a]pyridine-6-carboxylic acid methyl ester in 50 ml toluene was added 1 ml (10 mmol) benzaldehyde and the reaction refluxed (Dean-Stark apparatus) for 6h under azeotropic removal of water. The solution was concentrated in vacuo and the residue crystallized from n-hexane to afford 2.7 g (98%) of the title compound as a yellow solid. m.p. 170°-172° C.

### G. 2,3-Dimethyl-8-(1-phenyl-allylamino)-imidazo[1,2-a]pyridine-6-carboxylic acid methyl ester

A solution of 1.2 g (3.9 mmol) 8-(benzylidene-amino)-2,3-dimethyl-imidazo[1,2-*a*]pyridine-6-carboxylic acid methyl ester in 50 ml dichloromethane was cooled to -60° C and 11.7 ml (11.7 mmol) vinylmagnesium-bromide (1M in THF) were added dropwise. After 3h, the solution was poured into 20 ml saturated aqueous ammonium chloride solution and extracted with dichloromethane (3 x 200 ml). The organic layers were dried over magnesium sulphate and concentrated in vacuo. The residue was purified by chromatography on silica gel (toluene/dioxane = 10:1) to afford 1.1 g (91%) of the title compound as beige solid. m.p. 139°-140° C.

### H. 8-(bis-tert-Butyloxycarbonylamino)-2,3-dimethyl-7-((E)-3-phenyl-allyl)-imidazo[1,2-a]pyridine-6-carboxylic acid methylamide

To a solution of 0.4 g (0.78 mmol) 8-(bis-*tert*-butyloxycarbonylamino)-2,3-dimethyl-7-((E)-3-phenyl-allyl)-imidazo[1,2-a]pyridine-6-carboxylic acid in 20 ml dichloromethane were added 0.36 g (1.17 mmol) O-(1H-benzotriazol-1-yl)-N,N,N',N'-tetramethyluronium tetrafluoroborate (TBTU) and the reaction was stirred for 5 h at room temperature. 1.2 ml (1.8 mmol) Methylamine were added and the mixture was stirred for 12h at room temperature. The solvent was removed in vacuo and the residue was purified by column chromatography on silica gel (dichloromethane/methanol = 9:1) to afford 0.26 g (62%) of the title compound as a yellow oil.

### I. 8-(bis-tert-Butyloxycarbonylamino)-2,3-dimethyl-7-((E)-3-phenyl-allyl)-imidazo[1,2-a]pyridine-6-carboxylic acid dimethylamide

To a solution of 0.4 g (0.78 mmol) 8-(bis-*tert*-butyloxycarbonylamino)-2,3-dimethyl-7-((E)-3-phenyl-allyl)-imidazo[1,2-a]pyridine-6-carboxylic acid in 20 ml dichloromethane were added 0.36 g (1.17 mmol) O-(1H-benzotriazol-1-yl)-N,N,N',N'-tetramethyluronium tetrafluoroborate (TBTU) and the reaction was stirred for 5 h at room temperature. 1.1 ml (1.8 mmol) Dimethylamine were added and the mixture was stirred for 12h at room temperature. The solvent was removed in vacuo and the residue was purified by column chromatography on silica gel (dichloromethane/methanol = 9:1) to afford 0.3 g (63%) of the title compound as a yellow oil.

### J. 8-(bis-tert-Butyloxycarbonylamino)-2,3-dimethyl-7-((E)-3-phenyl-allyl)-imidazo[1,2-a]pyridine-6-carboxylic acid (2-hydroxy-ethyl)-amide

To a solution of 0.2 g (0.5 mmol) 8-(bis-*tert*-butyloxycarbonylamino)-2,3-dimethyl-7-((E)-3-phenyl-allyl)-imidazo[1,2-a]pyridine-6-carboxylic acid in 10 ml dichloromethane were added 0.23 g (0.7 mmol) O-(1H-benzotriazol-1-yl)-N,N,N',N'-tetramethyluronium tetrafluoroborate (TBTU) and the reaction was stirred for 5 h at room temperature. 0.08 g (0.9 mmol) Ethanolamine were added and the mixture was stirred for 12h at room temperature. The solvent was removed in vacuo and the residue was purified by column chromatography on silica gel (dichloromethane/methanol = 9:1) to afford 0.18 g (70%) of the title compound as a yellow oil.

### K. 1-[8-(bis-tert-Butyloxycarbonylamino)-2,3-dimethyl-7-((E)-3-phenyl-allyl)-imidazo[1,2-a]pyridin-6-y)]-1-pyrrolidin-1-yl-methanone

To a solution of 0.5 g (0.9 mmol) 8-(bis-*tert*-butyloxycarbonylamino)-2,3-dimethyl-7-((E)-3-phenyl-allyl)-imidazo[1,2-a]pyridine-6-carboxylic acid in 10 ml dichloromethane were added 0.47 g (1.4 mmol) O-(1 H-benzotriazol-1-yl)-N,N,N',N'-tetramethyluronium tetrafluoroborate (TBTU) and the reaction was stirred for 12h at room temperature. 0.13 g (1.8 mmol) Pyrrolidine were added and the mixture was stirred for 3 h at room temperature. The solvent was removed in vacuo and the residue was purified by column chromatography on silica gel (dichloromethane/methanol = 9:1) to afford 0.5 g (90%) of the title compound as a yellow oil.

### L. 8-(bis-tert-Butyloxycarbonylamino)-2,3-dimethyl-7-((E)-3-phenyl-allyl)-imidazo[1,2-a]pyridine-6-carboxylic acid (2-methoxy-ethyl)-amide

To a solution of 0.5 g (0.95 mmol) 8-(bis-*tert*-butyloxycarbonylamino)-2,3-dimethyl-7-((E)-3-phenyl-allyl)-imidazo[1,2-a]pyridine-6-carboxylic acid in 10 ml dichloromethane were added 0.47 g (1.4 mmol) O-(1 H-benzotriazol-1-yl)-N,N,N',N'-tetramethyluronium tetrafluoroborate (TBTU) and the reaction was stirred for 12h at room temperature. 0.14 g (1.8 mmol) 2-Methoxy-ethanolamine were added and the mixture was stirred for 3 h at room temperature. The solvent was removed in vacuo and the residue was purified by column chromatography on silica gel (dichloromethane/methanol = 9:1) to afford 0.5 g (89%) of the title compound as a yellow oil.

### M. 8-(bis-tert-Butyloxycarbonylamino)-2,3-dimethyl-7-((E)-3-phenyl-allyl)-imidazo[1,2-a]pyridine-6-carboxylic acid amide

To a solution of 0.4 g (0.78 mmol) 8-(bis-*tert*-butyloxycarbonylamino)-2,3-dimethyl-7-((E)-3-phenyl-allyl)-imidazo[1,2-a]pyridine-6-carboxylic acid in 20 ml dichloromethane were added 0.36 g (1.17 mmol) O-(1H-benzotriazol-1-yl)-N,N,N',N'-tetramethyluronium tetrafluoroborate (TBTU) and the reaction was stirred for 5 h at room temperature. 8 ml (1.8 mmol) Ammonia were added and the mixture was stirred for 12h at room temperature. The solvent was removed in vacuo and the residue was purified by column chromatography on silica gel (dichloromethane/methanol = 9:1) to afford 0.3 g (71%) of the title compound as a yellow oil.

### N. 8-(bis-tert-Butyloxycarbonylamino)-2,3-dimethyl-7-((E)-3-phenyl-allyl)-imidazo[1,2-a]pyridine-6-carboxylic acid

To a suspension of 5.5 g (10.2 mmol) 8-(bis-*tert*-butyloxycarbonylamino)-2,3-dimethyl-7-((E)-3-phenyl-allyl)-imidazo[1,2-a]pyridine-6-carboxylic acid methyl ester in dioxane (70 ml) was added a solution of 1.5 g (6.1 mmol) lithium hydroxide in 15 ml water and the reaction was refluxed for 3 h. After cooling to room temperature, the pH was adjusted to pH = 4 by adding 2N HCl solution and the reaction mixture was concentrated in vacuo. The residue was suspended in 100 ml water and extracted with dichloromethane (3 x 100 ml). The organic layers were dried over magnesium sulfate and concentrated in vacuo. The residue was crystallized from diethyl ether to afford 4 g (80%) of the title compound as a white solid. m.p. 224°-228° C

### O. 8-(bis-tert-Butyloxycarbonylamino)-2,3-dimethyl-7-((E)-3-phenyl-allyl)-imidazo(1,2-a]pyridine-6-carboxylic acid methyl ester

A solution of 3.4 g (11.9 mmol) 8-amino-2,3-dimethyl-7-((E)-3-phenyl-allyl)-imidazo[1,2-a]pyridine-6-carboxylic acid methyl ester in 100 ml dichloromethane was treated with 3.1 g (14.2 mmol) Boc-anhydride and catalytic DMAP and the reaction mixture was refluxed for 8 h. After cooling to room temperature, the solvent was removed in vacuo. The residue was crystallized from diethyl ether to afford 5.5 g (91%) of the title compound as a white solid. m.p. 199°-201° C.

### Commercial utility

The synthesis of the compounds of the formula 1 and of the formula 2 as described above offers an efficient access to the compounds of the formula 2 and can be used to synthesize a great variety of compounds of the formula 2 with different substituents R1, R2, R3 and Arom. These reaction sequences are also useful and applicable for the synthesis of compounds of the formula 2 on a larger scale.

The compounds of the formula 2 and their salts have valuable pharmacological properties which make them commercially utilizable. In particular, they exhibit marked inhibition of gastric acid secretion and an excellent gastric and intestinal protective action in warm-blooded animals, in particular humans. In this connection, the compounds according to the invention are distinguished by a high selectivity of action, an advantageous duration of action, a particularly good enteral activity, the absence of significant side effects and a large therapeutic range.

"Gastric and intestinal protection" in this connection is understood as meaning the prevention and treatment of gastrointestinal diseases, in particular of gastrointestinal inflammatory diseases and lesions (such as, for example, gastric ulcer, peptic ulcer, including peptic ulcer bleeding, duodenal ulcer, gastritis, hyperacidic or medicament-related functional dyspepsia), which can be caused, for example, by microorganisms (e.g. Helicobacter pylori), bacterial toxins, medicaments (e.g. certain antiinflammatories and antirheumatics, such as NSAIDs and COX-inhibitors), chemicals (e.g. ethanol), gastric acid or stress situations. "Gastric and intestinal protection" is understood to include, according to general knowledge, gastroesophageal reflux disease (GERD), the symptoms of which include, but are not limited to, heartburn and/or acid regurgitation.

In their excellent properties, the compounds of the formula 2 according to the invention surprisingly prove to be clearly superior to the compounds known from the prior art in various models in which the antiulcerogenic and the antisecretory properties are determined. On account of these properties, the compounds of the formula 2 and their pharmacologically acceptable salts are outstandingly suitable for use in human and veterinary medicine, where they are used, in particular, for the treatment and/or prophylaxis of disorders of the stomach and/or intestine.

A further subject of the invention are therefore the compounds of the formula 2 according to the invention for use in the treatment and/or prophylaxis of the abovementioned diseases.

The invention likewise includes the use of the compounds of the formula 2 according to the invention for the production of medicaments which are employed for the treatment and/or prophylaxis of the abovementioned diseases.

The invention furthermore includes the use of the compounds of the formula 2 according to the invention for the treatment and/or prophylaxis of the abovementioned diseases.

A further subject of the invention are medicaments which comprise one or more compounds of the formula 2 and/or their pharmacologically acceptable salts.

The medicaments are prepared by processes which are known per se and familiar to the person skilled in the art. As medicaments, the pharmacologically active compounds of the formula 2 according to the invention (= active compounds) are either employed as such, or preferably in combination with suitable pharmaceutical auxiliaries or excipients in the form of tablets, coated tablets, capsules, suppositories, patches (e.g. as TTS), emulsions, suspensions or solutions, the active compound content advantageously being between 0.1 and 95% and it being possible to obtain a pharmaceutical administration form exactly adapted to the active compound and/or to the desired onset and/or duration of action (e.g. a sustained-release form or an enteric form) by means of the appropriate selection of the auxiliaries and excipients.

The auxiliaries and excipients which are suitable for the desired pharmaceutical formulations are known to the person skilled in the art on the basis of his/her expert knowledge. In addition to solvents, gel-forming agents, suppository bases, tablet auxiliaries and other active compound excipients, it is possible to use, for example, antioxidants, dispersants, emulsifiers, antifoams, flavor corrigents, preservatives, solubilizers, colorants or, in particular, permeation promoters and complexing agents (e.g. cydodextrins).

The active compounds can be administered orally, parenterally or percutaneously.

In general, it has proven advantageous in human medicine to administer the active compound(s) in the case of oral administration in a daily dose of approximately 0.01 to approximately 20, preferably 0.05 to 5, in particular 0.1 to 1.5, mg/kg of body weight, if appropriate in the form of several, preferably 1 to 4, individual doses to achieve the desired result In the case of a parenteral treatment, similar or (in particular in the case of the intravenous administration of the active compounds), as a rule, lower doses can be used. The establishment of the optimal dose and manner of administration of the active compounds necessary in each case can easily be carried out by any person skilled in the art on the basis of his/her expert knowledge.

If the compounds of the formula 2 according to the invention and/or their salts are to be used for the treatment of the abovementioned diseases, the pharmaceutical preparations can also contain one or more pharmacologically active constituents of other groups of medicaments, for example: tranquillizers (for example from the group of the benzodiazepines, for example diazepam), spasmolytics (for example, bietamiverine or camylofine), anticholinergics (for example, oxyphencyclimine or phencarbamide), local anesthetics, (for example, tetracaine or procaine), and, if appropriate, also enzymes, vitamins or amino acids.

To be emphasized in this connection is in particular the combination of the compounds of the formula 2 according to the invention with pharmaceuticals which inhibit acid secretion, such as, for example, H₂ blockers (e.g. cimetidine, ranitidine), H⁺/K⁺ ATPase inhibitors (e.g. omeprazole, pantoprazole), or further with so-called peripheral anticholinergics (e.g. pirenzepine, telenzepine) and with gastrin antagonists with the aim of increasing the principal action in an additive or super-additive sense and/or of eliminating or of decreasing the side effects, or further the combination with antibacterially active substances (such as, for example, cephalosporins, tetracyclines, penicillins, macrolides, nitroimidazoles or alternatively bismuth salts) for the control of Helicobacter pylori. Suitable antibacterial co-components which may be mentioned are, for example, mezlocillin, ampicillin, amoxicillin, cefalothin, cefoxitin, cefotaxime, imipenem, gentamycin, amikacin, erythromycin, ciprofloxacin, metronidazole, clarithromycin, azithromycin and combinations thereof (for example clarithromycin + metronidazole).

In view of their excellent gastric and intestinal protection action, the compounds of formula 2 are suited for a free or fixed combination with those medicaments (e.g. certain antiinflammatories and antirheumatics, such as NSAIDs), which are known to have a certain ulcerogenic potency. In addition, the compounds of formula 2 are suited for a free or fixed combination with motility-modifying drugs.

### Pharmacology

The excellent gastric protective action and the gastric acid secretion-inhibiting action of the compounds of the formula 2 according to the invention can be demonstrated in investigations on animal experimental models. The compounds of the formula 2 according to the invention investigated in the model mentioned below have been provided with letters which correspond to the letters of these compounds in the examples.

### Testing of the secretion-inhibiting action on the perfused rat stomach

In Table A which follows, the influence of the compounds of the formula 2 according to the invention on the pentagastrin-stimulated acid secretion of the perfused rat stomach after intraduodenal administration in vivo is shown.

**Table A**

| No. | Dose (µmol/kg) i.d. | Inhibition of acid secretion (%) |
|---|---|---|
| **c** | 1.0 | > 50 |
| **e** | 1.0 | > 50 |
| **f** | 1.0 | > 50 |
| **h** | 1.0 | > 50 |
| **j** | 1.0 | > 50 |
| **k** | 1.0 | < 50 |
| **l** | 1.0 | < 50 |
| **m** | 1.0 | > 50 |
| **n** | 1.0 | < 50 |
| **o** | 1.0 | > 50 |
| **p** | 1.0 | < 50 |
| **q** | 1.0 | >50 |

### Methodology

The abdomen of anesthetized rats (CD rat, female, 200-250 g; 1.5 g/kg i.m. urethane) was opened after tracheotomy by a median upper abdominal incision and a PVC catheter was fixed transorally in the esophagus and another via the pylorus such that the ends of the tubes just projected into the gastric lumen. The catheter leading from the pylorus led outward into the right abdominal wall through a side opening.

After thorough rinsing (about 50-100 ml), warm (37°C) physiological NaCl solution was continuously passed through the stomach (0.5 ml/min, pH 6.8-6.9; Braun-Unita I). The pH (pH meter 632, glass electrode EA 147; φ = 5 mm, Metrohm) and, by titration with a freshly prepared 0.01 N NaOH solution to pH 7 (Dosimat 665 Metrohm), the secreted HCl were determined in the effluent in each case collected at an interval of 15 minutes.

The gastric secretion was stimulated by continuous infusion of 1 µg/kg (=1.65 ml/h) of i.v. pentagastrin (left femoral vein) about 30 min after the end of the operation (i.e. after determination of 2 preliminary fractions). The substances to be tested were administered intraduodenally in a 2.5 ml/kg liquid volume 60 min after the start of the continuous pentagastrin infusion.

The body temperature of the animals was kept at a constant 37.8-38°C by infrared irradiation and heat pads (automatic, stepless control by means of a rectal temperature sensor).

## Claims

1. A compound of the formula 1 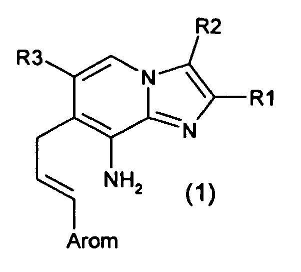 where
R1 is hydrogen, 1-4C-alkyl, 3-7C-cycloalkyl, 3-7C-cycloalkyl-1-4C-alkyl, 1-4C-alkoxy-1-4C-alkyl, 1-4C-alkoxycarbonyl, 2-4C-alkenyl, fluoro-1-4C-alkyl or hydroxy-1-4C-alkyl,
R2 is hydrogen, 1-4C-alkyl, 3-7C-cycloalkyl, 3-7C-cycloalkyl-1-4C-alkyl, 1-4C-alkoxycarbonyl, hydroxy-1-4C-alkyl, halogen, 2-4C-alkenyl, 2-4C-alkynyl, fluoro-1-4C-alkyl or cyanomethyl,
R3 is halogen, hydroxy-1-4C-alkyl, 1-4C-alkoxy-1-4C-alkyl, 1-4C-alkoxy-1-4C-alkoxy-1-4C-alkyl, 1-4C-alkoxycarbonyl, fluoro-1-4C-alkoxy-1-4C-alkyl or the radical -CO-NR31 R32,
where
R31 is hydrogen, 1-7C-alkyl, hydroxy-1-4C-alkyl, 3-7C-cycloalkyl, 1-4C-alkoxy-1-4C-alkyl and
R32 is hydrogen, 1-7C-alkyl, hydroxy-1-4C-alkyl, 3-7C-cycloalkyl or 1-4C-alkoxy-1-4C-alkyl,
or where
R31 and R32 together and including the nitrogen atom to which they are attached form a pyrrolidino, piperidino or morpholino radical
Arom is a R4-, R5-, R6- and R7-substituted mono- or bicyclic aromatic radical selected from the group consisting of phenyl, naphthyl, pyrrolyl, pyrazolyl, imidazolyl, 1,2,3-triazolyl, indolyl, benzimidazolyl, furanyl (furyl), benzofuranyl (benzofuryl), thiophenyl (thienyl), benzothiophenyl (benzothienyl), thiazolyl, isoxazolyl, pyridinyl, pyrimidinyl, quinolinyl and isoquinolinyl,
where
R4 is hydrogen, 1-4C-alkyl, hydroxy-1-4C-alkyl, 1-4C-alkoxy, 2-4C-alkenyloxy, carboxyl, 1-4C-alkoxycarbonyl, carboxy-1-4C-alkyl, halogen, hydroxyl, aryl, aryl-1-4C-alkyl, aryloxy, aryl-1-4C-alkoxy, trifluoromethyl, nitro, amino, mono- or di-1-4C-alkylamino or sulfonyl,
R5 is hydrogen, 1-4C-alkyl, 1-4C-alkoxy, 1-4C-alkoxycarbonyl, halogen, trifluoromethyl or hydroxyl,
R6 is hydrogen, 1-4C-alkyl or halogen and
R7 is hydrogen, 1-4C-alkyl or halogen,
where
aryl is phenyl or substituted phenyl having one, two or three identical or different substituents from the group consisting of 1-4C-alkyl, 1-4C-alkoxy, carboxyl, halogen, trifluoromethyl, nitro, trifluoromethoxy, hydroxyl and cyano,
and its salts.

2. A compound of the formula 1 as claimed in claim 1, in which
R1 is 1-4C-alkyl or 3-7C-cycloalkyl
R2 is hydrogen, 1-4C-alkyl, halogen or fluoro-1-4C-alkyl
R3 is halogen, hydroxy-1-4C-alkyl, 1-4C-alkoxy-1-4C-alkyl, 1-4C-alkoxy-1-4C-alkoxy-1-4C-alkyl, 1-4C-alkoxycarbonyl, or the radical -CO-NR31 R32,
where
R31 is hydrogen, 1-7C-alkyl, hydroxy-1-4C-alkyl, 3-7C-cycloalkyl or 1-4C-alkoxy-1-4C-alkyl and
R32 is hydrogen, 1-7C-alkyl, hydroxy-1-4C-alkyl, 3-7C-cycloalkyl or 1-4C-alkoxy-1-4C-alkyl,
or where
R31 and R32 together and including the nitrogen atom to which they are attached form a pyrrolidino, piperidino or morpholino radical
Arom is a R4-, R5-, R6- and R7-substituted phenyl,
where
R4 is hydrogen or 1-4C-alkyl,
R5 is hydrogen or 1-4C-alkyl,
R6 is hydrogen or 1-4C-alkyl and
R7 is hydrogen,
and its salts.

3. A compound of the formula 1 as claimed in claim 1, in which
R1 is 1-4C-alkyl,
R2 is 1-4C-alkyl,
R3 is halogen, 1-4C-alkoxycarbonyl, or the radical -CO-NR31 R32,
where
R31 is hydrogen, 1-4C-alkyl, hydroxy-1-4C-alkyl or 1-4C-alkoxy-1-4C-alkyl and
R32 is hydrogen, 1-4C-alkyl, hydroxy-1-4C-alkyl or 1-4C-alkoxy-1-4C-alkyl,
or where
R31 and R32 together and including the nitrogen atom to which they are attached form a pyrrolidino radical
Arom is phenyl,
and its salts.

4. A compound of the formula 1 as claimed in claim 1, in which
R1 is 1-4C-alkyl,
R2 is 1-4C-alkyl,
R3 is halogen or 1-4C-alkoxycarbonyl,
Arom is phenyl,
and its salts.

5. A compound of the formula 2 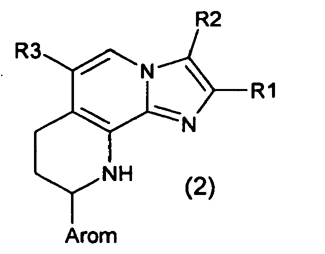 selected from the group consisting of
2,3-Dimethyl-8-phenyl-6,7,8,9-tetrahydro-1,3a,9-triaza-cyclopenta[a]naphthalene-5-carboxylic acid-(2-methoxy-ethyl)-amide,
2,3-Dimethyl-8-phenyl-6,7,8,9-tetrahydro-1,3a,9-triaza-cyclopenta[a]naphthalene-5-carboxylic Acid Ethyl Ester,
2,3-Dimethyl-8-phenyl-6,7,8,9-tetrahydro-1,3a,9-triaza-cyclopenta[a]naphthalene-5-carboxylic Acid Methylamide,
2,3-Dimethyl-8-phenyl-6,7,8,9-tetrahydro-1,3a,9-triaza-cyclopenta[a]naphthalene-5-carboxylic Acid (2-Hydroxy-ethyl)-amide,
2,3-Dimethyl-8-phenyl-6,7,8,9-tetrahydro-1,3a,9-triaza-cyclopenta[a]naphthalene-5-carboxylic Acid Dimethylamide
2,3-Dimethyl-8-phenyl-6,7,8,9-tetrahydro-1,3a,9-triaza-cyclopenta[a]naphthalene-5-carboxylic Acid Amide
1-(2,3-Dimethyl-8-phenyl-6,7,8,9-tetrahydro-1,3a,9-triaza-cyclopenta[a]naphthalen-5-yl)-1-morpholin-4-yl-methanone,
1-(2,3-Dimethyl-8-phenyl-6,7,8,9-tetrahydro-1,3a,9-triaza-cyclopenta[a]naphthalen-5-yl)-1-pyrrolidin-1-yl-methanone,
(2,3-Dimethyl-8-phenyl-6,7,8,9-tetrahydro-1,3a,9-triaza-cyclopenta[a]naphthalen-5-yl)-methanol,
2,3-Dimethyl-8-phenyl-6,7,8,9-tetrahydro-1,3a,9-triaza-cyclopenta[a]naphthalene-5-carboxylic Acid Cyclopropylamide,
and its salts.

6. A compound of the formula 2a 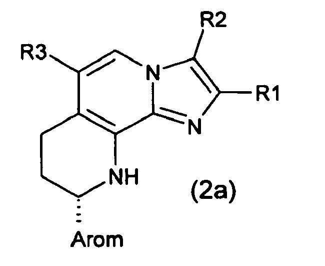 in which
R1 is hydrogen, 1-4C-alkyl, 3-7C-cycloalkyl, 3-7C-cycloalkyl-1-4C-alkyl, 1-4C-alkoxy-1-4C-alkyl, 1-4C-alkoxycarbonyl, 2-4C-alkenyl, fluoro-1-4C-alkyl or hydroxy-1-4C-alkyl,
R2 is hydrogen, 1-4C-alkyl, 3-7C-cycloalkyl, 3-7C-cycloalkyl-1-4C-alkyl, 1-4C-alkoxycarbonyl, hydroxy-1-4C-alkyl, halogen, 2-4C-alkenyl, 2-4C-alkynyl, fluoro-1-4C-alkyl or cyanomethyl,
R3 is halogen, hydroxy-1-4C-alkyl, 1-4C-alkoxy-1-4C-alkyl, 1-4C-alkoxy-1-4C-alkoxy-1-4C-alkyl, 1-4C-alkoxycarbonyl, fluoro-1-4C-alkoxy-1-4C-alkyl or the radical-CO-NR31R32,
where
R31 is hydrogen, 1-7C-alkyl, hydroxy-1-4C-alkyl, 3-7C-cycloalkyl, 1-4C-alkoxy-1-4C-alkyl and
R32 is hydrogen, 1-7C-alkyl, hydroxy-1-4C-alkyl, 3-7C-cycloalkyl or 1-4C-alkoxy-1-4C-alkyl,
or where
R31 and R32 together and including the nitrogen atom to which they are attached form a pyrrolidino, piperidino or morpholino radical
Arom is a R4-, R5-, R6- and R7-substituted mono- or bicyclic aromatic radical selected from the group consisting of phenyl, naphthyl, pyrrolyl, pyrazolyl, imidazolyl, 1,2,3-triazolyl, indolyl, benzimidazolyl, furanyl (furyl), benzofuranyl (benzofuryl), thiophenyl (thienyl), benzothiophenyl (benzothienyl), thiazolyl, isoxazolyl, pyridinyl, pyrimidinyl, quinolinyl and isoquinolinyl,
where
R4 is hydrogen, 1-4C-alkyl, hydroxy-1-4C-alkyl, 1-4C-alkoxy, 2-4C-alkenyloxy, carboxyl, 1-4C-alkoxycarbonyl, carboxy-1-4C-alkyl, halogen, hydroxyl, aryl, aryl-1-4C-alkyl, aryloxy, aryl-1-4C-alkoxy, trifluoromethyl, nitro, amino, mono- or di-1-4C-alkylamino or sulfonyl,
R5 is hydrogen, 1-4C-alkyl, 1-4C-alkoxy, 1-4C-alkoxycarbonyl, halogen, trifluoromethyl or hydroxyl,
R6 is hydrogen, 1-4C-alkyl or halogen and
R7 is hydrogen, 1-4C-alkyl or halogen,
where
aryl is phenyl or substituted phenyl having one, two or three identical or different substituents from the group consisting of 1-4C-alkyl, 1-4C-alkoxy, carboxyl, halogen, trifluoromethyl, nitro, trifluoromethoxy, hydroxyl and cyano,
and its salts.

7. A compound of the formula 2a as claimed in claim 6, in which
R1 is 1-4C-alkyl or 3-7C-cycloalkyl
R2 is hydrogen, 1-4C-alkyl, halogen or fluoro-1-4C-alkyl
R3 is halogen, hydroxy-1-4C-alkyl, 1-4C-alkoxy-1-4C-alkyl, 1-4C-alkoxy-1-4C-alkoxy-1-4C-alkyl, 1-4C-alkoxycarbonyl, or the radical -CO-NR31 R32,
where
R31 is hydrogen, 1-7C-alkyl, hydroxy-1-4C-alkyl, 3-7C-cycloalkyl or 1-4C-alkoxy-1-4C-alkyl and
R32 is hydrogen, 1-7C-alkyl, hydroxy-1-4C-alkyl, 3-7C-cycloalkyl or 1-4C-alkoxy-1-4C-alkyl,
or where
R31 and R32 together and including the nitrogen atom to which they are attached form a pyrrolidino, piperidino or morpholino radical
Arom is a R4-, R5-, R6- and R7-substituted phenyl,
where
R4 is hydrogen or 1-4C-alkyl,
R5 is hydrogen or 1-4C-alkyl,
R6 is hydrogen or 1-4C-alkyl and
R7 is hydrogen,
and its salts.

8. A compound of the formula 2a as claimed in claim 6, in which
R1 is 1-4C-alkyl,
R2 is 1-4C-alkyl,
R3 is halogen, 1-4C-alkoxycarbonyl, or the radical -CO-NR31 R32,
where
R31 is hydrogen, 1-4C-alkyl, hydroxy-1-4C-alkyl or 1-4C-alkoxy-1-4C-alkyl and
R32 is hydrogen, 1-4C-alkyl, hydroxy-1-4C-alkyl or 1-4C-alkoxy-1-4C-alkyl,
or where
R31 and R32 together and including the nitrogen atom to which they are attached form a pyrrolidino, piperidino or morpholino radical
Arom is phenyl,
and its salts.

9. A compound of the formula 2a 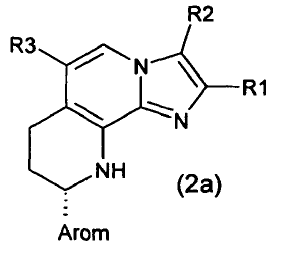 selected from the group consisting of
(8S)-2,3-Dimethyl-8-phenyl-6,7,8,9-tetrahydro-1,3a,9-triaza-cyclopenta[a]naphthalene-5-carboxylic acid-(2-methoxy-ethyl)-amide,
(8S)-2,3-Dimethyl-8-phenyl-6,7,8,9-tetrahydro-1,3a,9-triaza-cyclopenta[a]naphthalene-5-carboxylic Acid Ethyl Ester,
(8S)-2,3-Dimethyl-8-phenyl-6,7,8,9-tetrahydro-1,3a,9-triaza-cyclopenta[a]naphthalene-5-carboxylic Acid Methylamide,
(8S)-2,3-Dimethyl-8-phenyl-6,7,8,9-tetrahydro-1,3a,9-triaza-cyclopenta[a]naphthalene-5-carboxylic Acid (2-Hydroxy-ethyl)-amide,
(8S)-2,3-Dimethyl-8-phenyl-6,7,8,9-tetrahydro-1,3a,9-triaza-cyclopenta[a]naphthalene-5-carboxytic Acid Dimethylamide
(8S)-2,3-Dimethyl-8-phenyl-6,7,8,9-tetrahydro-1,3a,9-triaza-cyclopenta[a]naphthalene-5-carboxylic Acid Amide
(8S)-1-(2,3-Dimethyl-8-phenyl-6,7,8,9-tetrahydro-1,3a,9-triaza-cyclopenta[a]naphthalen-5-yl)-1-morpholin-4-yl-methanone
(8S)-1-(2,3-Dimethyl-8-phenyl-6,7,8,9-tetrahydro-1,3a,9-triaza-cyclopenta[a]naphthalen-5-yl)-1-pyrrolidin-1-yl-methanone
(8S)-(2,3-Dimethyl-8-phenyl-6,7,8,9-tetrahydro-1,3a,9-triaza-cyclopenta[a]naphthalen-5-yl)-methanol
(8S)-2,3-Dimethyl-8-phenyl-6,7,8,9-tetrahydro-1,3a,9-triaza-cyclopenta[a]naphthalene-5-carboxylic Acid Cyclopropylamide
and its salts.

10. A process for the synthesis of a compound of the formula 2, 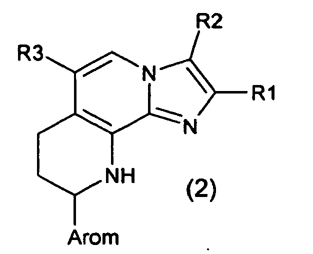 in which R1, R2, R3 and Arom have the meanings as in claim 1, which comprises the conversion of a compound of the formula 1 as claimed in claim 1 into a compound of the formula 2, followed ,if desired, by further derivatization of the resulting compound of the formula 2 into another compound of the formula 2.

11. A process for the synthesis of a compound of the formula 1 as claimed in claim 1, which comprises the conversion of a compound of the formula 5, in which R1, R2, R3 and Arom have the meanings as indicated in claim 1, into the corresponding compound of the formula 1, followed ,if desired, by further derivatization of the resulting compound of the formula 1 into another compound of the formula 1.

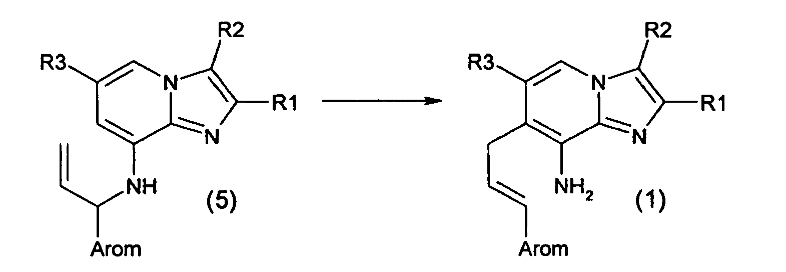

12. A medicament comprising a compound as claimed in any of claims 5 to 9 and/or a pharmacologically acceptable salt thereof together with customary pharmaceutical auxiliaries and/or excipients.

13. The use of a compound as claimed in any of claims 5 to 9 and/or its pharmacologically acceptable salts for the production of medicaments for the prevention and treatment of gastrointestinal disorders.

## Patentansprüche

1. Verbindungen der Formel 1 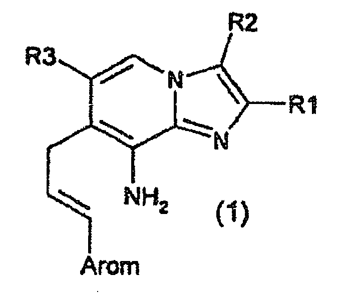 worin
R1 Wasserstoff, 1-4C-Alkyl, 3-7C-Cycloalkyl, 3-7C-Cycloalkyl-1-4C-alkyl, 1-4C-Alkoxy-1-4C-alkyl, 1-4C-Alkoxycarbonyl, 2-4C-Alkenyl, Fluor-1-4C-alkyl oder Hydroxy-1-4C-alkyl bedeutet,
R2 Wasserstoff, 1-4C-Alkyl, 3-7C-Cycloalkyl, 3-7C-Cycloalkyl-1-4C-alkyl, 1-4C-Alkoxycarbonyl, Hydroxy-1-4C-alkyl, Halogen, 2-4C-Alkenyl, 2-4C-Alkinyl, Fluor-1-4C-alkyl oder Cyanomethyl bedeutet,
R3 Halogen, Hydroxy-1-4C-alkyl, 1-4C-Alkoxy-1-4C-alkyl, 1-4C-Alkoxy-1-4C-alkoxy-1-4C-alkyl, 1-4C-Alkoxycarbonyl, Fluor-1-4C-alkoxy-1-4C-alkyl oder den Rest -CO-NR31R32 bedeutet,
wobei
R31 Wasserstoff, 1-7C-Alkyl, Hydroxy-1-4C-alkyl, 3-7C-Cycloalkyl, 1-4C-Alkoxy-1-4C-alkyl bedeutet und
R32 Wasserstoff, 1-7C-Alkyl, Hydroxy-1-4C-alkyl, 3-7C-Cycloalkyl oder 1-4C-Alkoxy-1-4C-alkyl bedeutet,
oder wobei
R31 und R32 gemeinsam unter Einschluß des Stickstoffatoms, an das beide gebunden sind, einen Pyrrolidino-, Piperidino- oder Morpholinorest darstellen.
Arom für einen durch R4, R5, R6 und R7 substituierten mono- oder bicyclischen aromatischen Rest steht, der ausgewählt ist aus der Gruppe bestehend aus Phenyl, Naphthyl, Pyrrolyl, Pyrazolyl, Imidazolyl, 1,2,3-Triazolyl, Indolyl, Benzimidazolyl, Furanyl (Furyl), Benzofuranyl (Benzofuryl), Thiophenyl (Thienyl), Benzothiophenyl (Benzothienyl), Thiazolyl, Isoxazolyl, Pyridinyl, Pyrimidinyl, Chinolinyl und Isochinolinyl, wobei
R4 Wasserstoff, 1-4C-Alkyl, Hydroxy-1-4C-alkyl, 1-4C-Alkoxy, 2-4C-Alkenyloxy, Carboxyl, 1-4C-Alkoxycarbonyl, Carboxy-1-4C-alkyl, Halogen, Hydroxyl, Aryl, Aryl-1-4C-alkyl, Aryloxy, Aryl-1-4C-alkoxy, Trifluormethyl, Nitro, Amino, Mono- oder Di-1-4C-alkylamino oder Sulfonyl bedeutet,
R5 Wasserstoff, 1-4C-Alkyl, 1-4C-Alkoxy, 1-4C-Alkoxycarbonyl, Halogen, Trifluormethyl oder Hydroxyl bedeutet,
R6 Wasserstoff, 1-4C-Alkyl oder Halogen bedeutet und
R7 Wasserstoff, 1-4C-Alkyl oder Halogen bedeutet,
wobei
Aryl Phenyl oder substituiertes Phenyl mit einem, zwei oder drei gleichen oder verschiedenen Substituenten bedeutet, aus der Gruppe bestehend aus 1-4C-Alkyl, 1-4C-Alkoxy, Carboxyl, Halogen, Trifluormethyl, Nitro, Trifluormethoxy, Hydroxyl und Cyano,
und ihre Salze.

2. Verbindungen der Formel 1 nach Anspruch 1, in denen
R1 1-4C-Alkyl oder 3-7C-Cycloalkyl bedeutet,
R2 Wasserstoff, 1-4C-Alkyl, Halogen oder Fluor-1-4C-alkyl bedeutet,
R3 Halogen, Hydroxy-1-4C-alkyl, 1-4C-Alkoxy-1-4C-alkyl, 1-4C-Alkoxy-1-4C-alkoxy-1-4C-alkyl, 1-4C-Alkoxycarbonyl oder den Rest -CO-NR31R32 bedeutet;
wobei
R31 Wasserstoff, 1-7C-Alkyl, Hydroxy-1-4C-alkyl, -3-7C-Cycloalkyl oder 1-4C-Alkoxy-1-4C-alkyl bedeutet, und
R32 Wasserstoff, 1-7C-Alkyl, Hydroxy-1-4C-alkyl, 3-7C-Cycloalkyl oder 1-4C-Alkoxy-1-4C-alkyl bedeutet,
oder wobei
R31 und R32 gemeinsam unter Einschluß des Stickstoffatoms, an das beide gebunden sind, einen Pyrrolidino-, Piperidino- oder Morpholinorest darstellen.
Arom für ein durch R4, R5, R6 und R7 substituiertes Phenyl steht,
wobei
R4 Wasserstoff oder 1-4C-Alkyl bedeutet,
R5 Wasserstoff oder 1-4C-Alkyl bedeutet,
R6 Wasserstoff oder 1-4C-Alkyl bedeutet und
R7 Wasserstoff bedeutet,
und ihre Salze.

3. Verbindungen der Formel 1 nach Anspruch 1, in denen
R1 1-4C-Alkyl bedeutet,
R2 1-4C-Alkyl bedeutet,
R3 Halogen, 1-4C-Alkoxycarbonyl oder den Rest -CO-NR31R32 bedeutet,
wobei
R31 Wasserstoff, 1-4C-Alkyl, Hydroxy-1-4C-alkyl oder 1-4C-Alkoxy-1-4C-alkyl bedeutet, und
R32 Wasserstoff, 1-4C-Alkyl, Hydroxy-1-4C-alkyl oder 1-4C-Alkoxy-1-4C-alkyl bedeutet,
oder wobei
R31 und R32 gemeinsam unter Einschluß des Stickstoffatoms, an das beide gebunden sind, einen Pyrrolidinorest darstellen.
Arom für Phenyl steht,
und ihre Salze.

4. Verbindungen der Formel 1 nach Anspruch 1, in denen
R1 1-4C-Alkyl bedeutet,
R2 1-4C-Alkyl bedeutet,
R3 Halogen oder 1-4C-Alkoxycarbonyl bedeutet,
Arom für Phenyl steht,
und ihre Salze.

5. Verbindungen der Formel 2 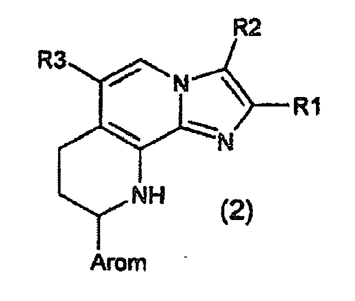 ausgewählt aus der Gruppe bestehend aus
2,3-Dimethyl-8-phenyl-6,7,8,9-tetrahydro-1,3a,9-triazacyclopenta[a]naphthalin-5-carbonsäure-(2-methoxyethyl)amid,
2,3-Dimethyl-8-phenyl-6,7,8,9-tetrahydro-1,3a,9-triazacyclopenta[a]naphthalin-5-carbonsäureethylester,
2,3-Dimethyl-8-phenyl-6,7,8,9-tetrahydro-1,3a,9-triazacyclopenta[a]naphthalin-5-carbonsäuremethylamid,
2,3-Dimethyl-8-phenyl-6,7,8,9-tetrahydro-1,3a,9-triazacyclopenta[a]naphthalin-5-carbonsäure-(2-hydroxyethyl)amid,
2,3-Dimethyl-8-phenyl-6,7,8,9-tetrahydro-1,3a,9-triazacyclopenta[a]naphthalin-5-carbonsäuredimethylamid,
2,3-Dimethyl-8-phenyl-6,7,8,9-tetrahydro-1,3a,9-triazacyclopenta[a]naphthalin-5-carbonsäureamid,
1-(2,3-Dimethyl-8-phenyl-6,7,8,9-tetrahydro-1,3a,9-triazacyclopenta[a]naphthalin-5-yl)-1-morpholin-4-yl-methanon,
1-(2,3-Dimethyl-8-phenyl-6,7,8,9-tetrahydro-1,3a,9-triazacyclopenta[a]naphthalin-5-yl)-1-pyrrolidin-1-yl-methanon,
(2,3-Dimethyl-8-phenyl-6,7,8,9-tetrahydro-1,3a,9-triazacyclopenta[a]naphthalin-5-yl)methanol,
2,3-Dimethyl-8-phenyl-6,7,8,9-tetrahydro-1,3a,9-triazacyclopenta[a]naphthalin-5-carbonsäurecyclopropylamid
und ihren Salzen.

6. Verbindungen der Formel 2a 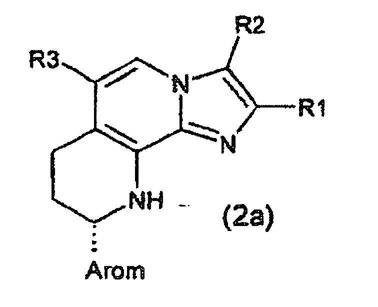 in denen
R1 Wasserstoff, 1-4C-Alkyl, 3-7C-Cycloalkyl, 3-7C-Cycloalkyl-1-4C-alkyl, 1-4C-Alkoxy-1-4C-alkyl, 1-4C-Alkoxycarbonyl, 2-4C-Alkenyl, Fluor-1-4C-alkyl oder Hydroxy-1-4C-alkyl bedeutet,
R2 Wasserstoff, 1-4C-Alkyl, 3-7C-Cycloalkyl, 3-7C-Cycloalkyl-1-4C-alkyl, 1-4C-Alkoxycarbonyl, Hydroxy-1-4C-alkyl, Halogen, 2-4C-Alkenyl, 2-4C-Alkinyl, Fluor-1-4C-alkyl oder Cyanomethyl bedeutet,
R3 Halogen, Hydroxy-1-4C-alkyl, 1-4C-Alkoxy-1-4C-alkyl, 1-4C-Alkoxy-1-4C-alkoxy-1-4C-alkyl, 1-4C-Alkoxycarbonyl, Fluor-1-4C-alkoxy-1-4C-alkyl oder den Rest -CO-NR31R32 bedeutet,
wobei
R31 Wasserstoff, 1-7C-Alkyl, Hydroxy-1-4C-alkyl, 3-7C-Cycloalkyl, 1-4C-Alkoxy-1-4C-alkyl bedeutet und
R32 Wasserstoff, 1-7C-Alkyl, Hydroxy-1-4C-alkyl, 3-7C-Cycloalkyl oder 1-4C-Alkoxy-1-4C-alkyl bedeutet,
oder wobei
R31 und R32 gemeinsam unter Einschluß des Stickstoffatoms, an das beide gebunden sind, einen Pyrrolidino-, Piperidino- oder Morpholinorest darstellen.
Arom für einen durch R4, R5, R6 und R7 substituierten mono- oder bicyclischen aromatischen Rest steht, der ausgewählt ist aus der Gruppe bestehend aus Phenyl, Naphthyl, Pyrrolyl, Pyrazolyl, Imidazolyl, 1,2,3-Triazolyl, Indolyl, Benzimidazolyl, Furanyl (Furyl), Benzofuranyl (Benzofuryl), Thiophenyl (Thienyl), Benzothiophenyl (Benzothienyl), Thiazolyl, Isoxazolyl, Pyridinyl, Pyrimidinyl, Chinolinyl und Isochinolinyl, wobei
R4 Wasserstoff, 1-4C-Alkyl, Hydroxy-1-4C-alkyl, 1-4C-Alkoxy, 2-4C-Alkenyloxy, Carboxyl, 1-4C-Alkoxycarbonyl, Carboxy-1-4C-alkyl, Halogen, Hydroxyl, Aryl, Aryl-1-4C-alkyl, Aryloxy, Aryl-1-4C-alkoxy, Trifluormethyl, Nitro, Amino, Mono- oder Di-1-4-alkylamino oder Sulfonyl bedeutet,
R5 Wasserstoff, 1-4C-Alkyl, 1-4C-Alkoxy, 1-4C-Alkoxycarbonyl, Halogen, Trifluormethyl oder Hydroxyl bedeutet,
R6 Wasserstoff, 1-4C-Alkyl oder Halogen bedeutet und
R7 Wasserstoff, 1-4C-Alkyl oder Halogen bedeutet,
wobei
Aryl Phenyl oder substituiertes Phenyl mit einem, zwei oder drei gleichen oder verschiedenen Substituenten bedeutet, aus der Gruppe bestehend aus 1-4C-Alkyl, 1-4C-Alkoxy, Carboxyl, Halogen, Trifluormethyl, Nitro, Trifluormethoxy, Hydroxyl und Cyano,
und ihre Salze.

7. Verbindungen der Formel 2a nach Anspruch 6, in denen
R1 1-4C-Alkyl oder 3-7C-Cycloalkyl bedeutet,
R2 Wasserstoff, 1-4C-Alkyl, Halogen oder Fluor-1-4C-alkyl bedeutet,
R3 Halogen, Hydroxy-1-4C-alkyl, 1-4C-Alkoxy-1-4C-alkyl, 1-4C-Alkoxy-1-4C-alkoxy-1-4C-alkyl, 1-4C-Alkoxycarbonyl oder den Rest -CO-NR31R32 bedeutet;
wobei
R31 Wasserstoff, 1-7C-Alkyl, Hydroxy-1-4C-alkyl, 3-7C-Cycloalkyl oder 1-4C-Alkoxy-1-4C-alkyl bedeutet, und
R32 Wasserstoff, 1-7C-Alkyl, Hydroxy-1-4C-alkyl, 3-7C-Cycloalkyl oder 1-4C-Alkoxy-1-4C-alkyl bedeutet,
oder wobei
R31 und R32 gemeinsam unter Einschluß des Stickstoffatoms, an das beide gebunden sind, einen Pyrrolidino-, Piperidino- oder Morpholinorest darstellen.
Arom für ein durch R4, R5, R6 und R7 substituiertes Phenyl steht,
wobei
R4 Wasserstoff oder 1-4C-Alkyl bedeutet,
R5 Wasserstoff oder 1-4C-Alkyl bedeutet,
R6 Wasserstoff oder 1-4C-Alkyl bedeutet und
R7 Wasserstoff bedeutet,
und ihre Salze.

8. Verbindungen der Formel 2a nach Anspruch 6, in denen
R1 1-4C-Alkyl bedeutet,
R2 1-4C-Alkyl bedeutet,
R3 Halogen, 1-4C-Alkoxycarbonyl oder den Rest -CO-NR31R32 bedeutet,
wobei
R31 Wasserstoff, 1-4C-Alkyl, Hydroxy-1-4C-alkyl oder 1-4C-Alkoxy-1-4C-alkyl bedeutet, und
R32 Wasserstoff, 1-4C-Alkyl, Hydroxy-1-4C-alkyl oder 1-4C-Alkoxy-1-4C-alkyl bedeutet,
oder wobei
R31 und R32 gemeinsam unter Einschluß des Stickstoffatoms, an das beide gebunden sind, einen Pyrrolidino-, Piperidino- oder Morpholino rest darstellen.
Arom für Phenyl steht,
und ihre Salze.

9. Verbindungen der Formel 2a 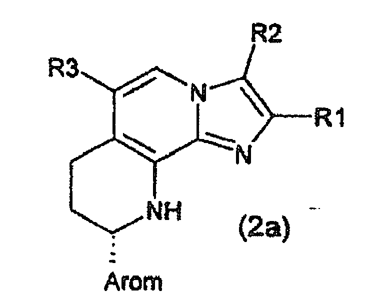 ausgewählt aus der Gruppe bestehend aus
(8S)-2,3-Dimethyl-8-phenyl-6,7,8,9-tetrahydro-1,3a,9-triazacyclopenta[a]naphthalin-5-carbonsäure-(2-methoxyethyl)amid,
(8S)-2,3-Dimethyl-8-phenyl-6,7,8,9-tetrahydro-1,3a,9-triazacyclopenta[a]naphthalin-5-carbonsäureethylester,
(8S)-2,3-Dimethyl-8-phenyl-6,7,8,9-tetrahydro-1,3a,9-triazacyclopenta[a]naphthalin-5-carbonsäuremethylamid,
(8S)-2,3-Dimethyl-8-phenyl-6,7,8,9-tetrahydro-1,3a,9-triazacyclopenta[a]naphthalin-5-carbonsäure-(2-hydroxyethyl)amid,
(8S)-2,3-Dimethyl-8-phenyl-6,7,8,9-tetrahydro-1,3a,9-triazacyclopenta[a]naphthalin-5-carbonsäuredimethylamid,
(8S)-2,3-Dimethyl-8-phenyl-6,7,8,9-tetrahydro-1,3a,9-triazacyclopenta[a]naphthalin-5-carbonsäureamid,
(8S)-1-(2,3-Dimethyl-8-phenyl-6,7,8,9-tetrahydro-1,3a,9-triazacyclopenta[a]naphthalin-5-yl)-1-morpholin-4-yl-methanon,
(8S)-1-(2,3-Dimethyl-8-phenyl-6,7,8,9-tetrahydro-1,3a,9-triazacyclopenta[a]naphthalin-5-yl)-1-pyrrolidon-1-yl-methanon
(8S)-(2,3-Dimethyl-8-phenyl-6,7,8,9-tetrahydro-1,3a,9-triazacyclopenta[a]naphthalin-5-yl)methanol,
(8S)-2,3-Dimethyl-8-phenyl-6,7,8,9-tetrahydro-1,3a,9-triazacyclopenta[a]naphthalin-5-carbonsäurecyclopropylamid
und ihren Salzen.

10. Verfahren zur Synthese einer Verbindung der Formel 2, 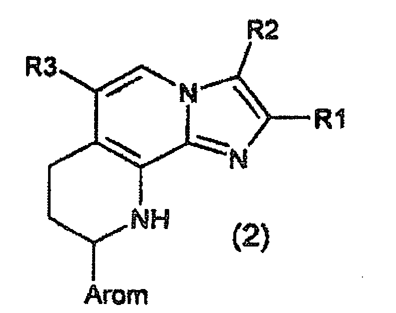 in welcher R1, R2, R3 und Arom die gleichen Bedeutungen wie in Anspruch 1 haben, bei dem man eine Verbindung der Formel 1 nach Anspruch 1 in eine Verbindung der Formel 2 umwandelt und anschließend, falls gewünscht, die auf diese Weise erhaltene Verbindung der Formel 2 weiter zu einer anderen Verbindung der Formel 2 derivatisiert.

11. Verfahren zur Synthese einer Verbindung der Formel 1 nach Anspruch 1, bei dem man eine Verbindung der Formel 5, in welcher R1, R2, R3 und Arom die in Anspruch 1 angegebenen Bedeutungen haben, in die entsprechende Verbindung der Formel 1 umwandelt und anschließend, falls gewünscht, die auf diese Weise erhaltene Verbindung der Formel 1 weiter zu einer anderen Verbindung der Formel 1 derivatisiert.

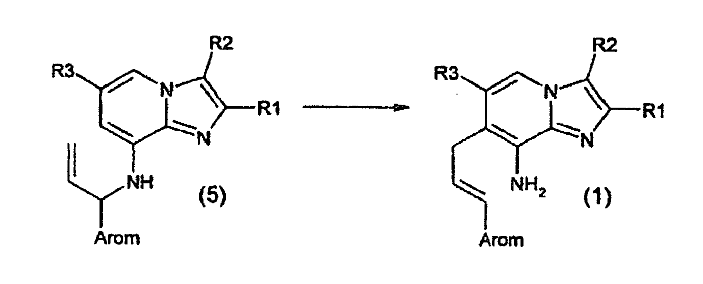

12. Arzneimittel, enthaltend eine Verbindung nach einem der Ansprüche 5 bis 9 und/oder ein pharmakologisch verträgliches Salz davon zusammen mit üblichen pharmazeutischen Hilfs- und/oder Trägerstoffen.

13. Verwendung von Verbindungen nach einem der Ansprüche 5 bis 9 und ihren pharmakologisch verträglichen Salzen zur Herstellung von Arzneimitteln zur Verhütung und Behandlung gastrointestinaler Krankheiten.

## Revendications

1. Composé de formule 1 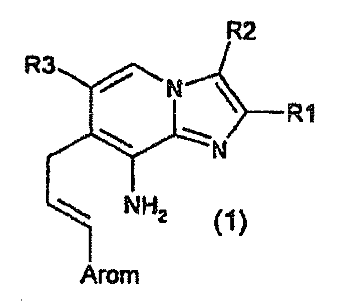 dans laquelle
R1 est hydrogène, 1-4C-alkyle, 3-7C-cycloalkyle, 3-7C-cycloalkyl-1-4C-alkyle, 1-4C-alcoxy-1-4C-alkyle, 1-4C-alcoxycarbonyle, 2-4C-alcényle, fluoro-1-4C-alkyle ou hydroxy-1-4C-alkyle,
R2 est hydrogène, 1-4C-alkyle, 3-7C-cycloalkyle, 3-7C-cycloalkyl-1-4C-alkyle, 1-4C-alcoxycarbonyle, hydroxy-1-4C-alkyle, halogène, 2-4C-alcényle, 2-4C-alcynyle, fluoro-1-4C-alkyle ou cyanométhyle,
R3 est halogène, hydroxy-1-4C-alkyle, 1-4C-alcoxy-1-4C-alkyle, 1-4C-alcoxy-1-4C-alcoxy-1-4C-alkyle, 1-4C-alcoxycarbonyle, fluoro-1-4C-alcoxy-1-4C-alkyle ou le radical -CO-NR31R32,
où
R31 est hydrogène, 1-7C-alkyle, hydroxy-1-4C-alkyle, 3-7C-cycloalkyle, 1-4C-alcoxy-1-4C-alkyle et
R32 est hydrogène, 1-7C-alkyle, hydroxy-1-4C-alkyle, 3-7C-cycloalkyle ou 1-4C-alcoxy-1-4C-alkyle,
ou où
R31 et R32, conjointement et y compris l'atome d'azote auquel ils sont liés, forment un radical pyrrolidino, pipéridino ou morpholino
Arom est un radical aromatique mono- ou bicyclique substitué par R4, R5, R6 et R7, choisi dans le groupe constitué de phényle, de naphtyle, de pyrrolyle, de pyrazolyle, d'imidazolyle, de 1,2,3-triazolyle, d'indolyle, de benzimidazolyle, de furanyle (furyle), de benzofuranyle (benzofuryle), de thiophényle (thiényle), de benzothiophényle (benzothiényl), de thiazolyle, d'isoxazolyle, de pyridinyle, de pyrimidinyle, de quinoléinyle et d'isoquinoléinyle, où
R4 est hydrogène, 1-4C-alkyle, hydroxy-1-4C-alkyle, 1-4C-alcoxy, 2-4C-alcényloxy, carboxyle, 1-4C-alcoxycarbonyle, carboxy-1-4C-alkyle, halogène, hydroxyle, aryle, aryl-1-4C-alkyle, aryloxy, aryl-1-4C-alcoxy, trifluorométhyle, nitro, amino, mono- ou di-1-4C-alkylamino ou sulfonyle,
R5 est hydrogène, 1-4C-alkyle, 1-4C-alcoxy, 1-4C-alcoxycarbonyle, halogène, trifluorométhyle ou hydroxyle,
R6 est hydrogène, 1-4C-alkyle ou halogène et
R7 est hydrogène, 1-4C-alkyle ou halogène,
où
aryle est phényle ou phényle substitué ayant un, deux ou trois substituants identiques ou différents provenant du groupe constitué de 1-4C-alkyle, de 1-4C-alcoxy, de carboxyle, d'halogène, de trifluorométhyle, de nitro, de trifluorométhoxy, d'hydroxyle et de cyano,
et ses sels.

2. Composé de formule 1 selon la revendication 1, **caractérisé en ce que**
R1 est 1-4C-alkyle ou 3-7C-cycloalkyle,
R2 est hydrogène, 1-4C-alkyle, halogène ou fluoro-1-4C-alkyle,
R3 est halogène, hydroxy-1-4C-alkyle, 1-4C-alcoxy-1-4C-alkyle, 1-4C-alcoxy-1-4C-alcoxy-1-4C-alkyle, 1-4C-alcoxycarbonyle ou le radical -CO-NR31R32,
où
R31 est hydrogène, 1-7C-alkyle, hydroxy-1-4C-alkyle, 3-7C-cycloalkyle ou 1-4C-alcoxy-1-4C-alkyle et
R32 est hydrogène, 1-7C-alkyle, hydroxy-1-4C-alkyle, 3-7C-cycloalkyle ou 1-4C-alcoxy-1-4C-alkyle,
ou où
R31 et R32, conjointement et y compris l'atome d'azote auquel ils sont liés, forment un radical pyrrolidino, pipéridino ou morpholino
Arom est un phényle substitué par R4, R5, R6 et R7,
où
R4 est hydrogène ou 1-4C-alkyle,
R5 est hydrogène ou 1-4C-alkyle,
R6 est hydrogène ou 1-4C-alkyle et
R7 est hydrogène,
et ses sels.

3. Composé de formule 1 selon la revendication 1, **caractérisé en ce que**
R1 est 1-4C-alkyle,
R2 est 1-4C-alkyle,
R3 est halogène, 1-4C-alcoxycarbonyle ou le radical -CO-NR31R32,
où
R31 est hydrogène, 1-4C-alkyle, hydroxy-1-4C-alkyle ou 1-4C-alcoxy-1-4C-alkyle et
R32 est hydrogène, 1-4C-alkyle, hydroxy-1-4C-alkyle ou 1-4C-alcoxy-1-4C-alkyle,
ou où
R31 et R32, conjointement et y compris l'atome d'azote auquel ils sont liés, forment un radical pyrrolidino
Arome est phényle,
et ses sels.

4. Composé de formule 1 selon la revendication 1, **caractérisé en ce que**
R1 est 1-4C-alkyle,
R2 est 1-4C-alkyle,
R3 est halogène ou 1-4C-alcoxycarbonyle,
Arom est phényle,
et ses sels.

5. Composé de formule 2 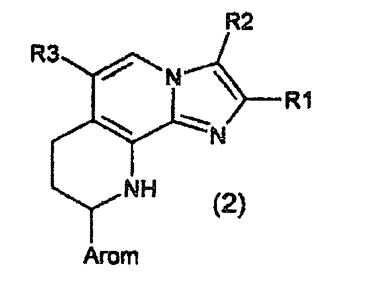 choisi dans le groupe constitué de
(2-méthoxyéthyl)amide d'acide 2,3-diméthyl-8-phényl-6,7,8,9-tétrahydro-1,3a,9-triazacyclopenta[a]naphtalène-5-carboxylique,
ester éthylique d'acide 2,3-diméthyl-8-phényl-6,7,8,9-tétrahydro-1,3a,9-triazacyclopenta[a]naphtalène-5-carboxylique,
méthylamide d'acide 2,3-diméthyl-8-phényl-6,7,8,9-tétrahydro-1,3a,9-triazacyclopenta[a]naphtalène-5-carboxylique,
(2-hydroxyéthyl)amide d'acide 2,3-diméthyl-8-phényl-6,7,8,9-tétrahydro-1,3a,9-triazacyclopenta[a]naphtalène-5-carboxylique,
diméthylamide d'acide 2,3-diméthyl-8-phényl-6,7,8,9-tétrahydro-1,3a,9-triazacyclopenta[a]naphtalène-5-carboxylique,
amide d'acide 2,3-diméthyl-8-phényl-6,7,8,9-tétrahydro-1,3a,9-triazacyclopenta[a]naphtalène-5-carboxylique,
1-(2,3-diméthyl-8-phényl-6,7,8,9-tétrahydro-1,3a,9-triazacyclopenta[a]naphtalén-5-yl)-1-morpholin-4-ylméthanone,
1-(2,3-diméthyl-8-phényl-6,7,8,9-tétrahydro-1,3a,9-triazacyclopenta[a]naphtalén-5-yl)-1-pyrrolidin-1-ylméthanone,
(2,3-diméthyl-8-phényl-6,7,8,9-tétrahydro-1,3a,9-triazacyclopenta[a]naphtalén-5-yl)méthanol,
cyclopropylamide d'acide 2,3-diméthyl-8-phényl-6,7,8,9-tétrahydro-1,3a,9-triazacyclopenta[a]naphtalène-5-carboxylique,
et ses sels.

6. Composé de formule 2a 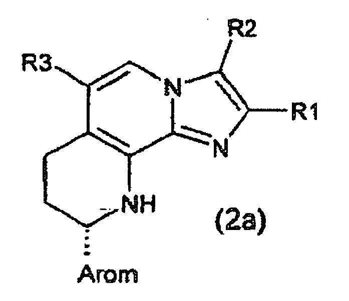 dans laquelle
R1 est hydrogène, 1-4C-alkyle, 3-7C-cycloalkyle, 3-7C-cycloalkyl-1-4C-alkyle, 1-4C-alcoxy-1-4C-alkyle, 1-4C-alcoxycarbonyle, 2-4C-alcényle, fluoro-1-4C-alkyle ou hydroxy-1-4C-alkyle,
R2 est hydrogène, 1-4C-alkyle, 3-7C-cycloalkyle, 3-7C-cycloalkyl-1-4C-alkyle, 1-4C-alcoxycarbonyle, hydroxy-1-4C-alkyle, halogène, 2-4C-alcényle, 2-4C-alcynyle, fluoro-1-4C-alkyle ou cyanométhyle,
R3 est halogène, hydroxy-1-4C-alkyle, 1-4C-alcoxy-1-4C-alkyle, 1-4C-alcoxy-1-4C-alcoxy-1-4C-alkyle, 1-4C-alcoxycarbonyle, fluoro-1-4C-alcoxy-1-4C-alkyle ou le radical -CO-NR31R32,
où
R31 est hydrogène, 1-7C-alkyle, hydroxy-1-4C-alkyle, 3-7C-cycloalkyle, 1-4C-alcoxy-1-4C-alkyle et
R32 est hydrogène, 1-7C-alkyle, hydroxy-1-4C-alkyle, 3-7C-cycloalkyle ou 1-4C-alcoxy-1-4C-alkyle,
ou où
R31 et R32, conjointement et y compris l'atome d'azote auquel ils sont liés, forment un radical pyrrolidino, pipéridino ou morpholino
Arom est un radical aromatique mono- ou bicyclique substitué par R4, R5, R6 et R7, choisi dans le groupe constitué de phényle, de naphtyle, de pyrrolyle, de pyrazolyle, d'imidazolyle, de 1,2,3-triazolyle, d'indolyle, de benzimidazolyle, de furanyle (furyle), de benzofuranyle (benzofuryle), de thiophényle (thiényle), de benzothiophényle (benzothiényl), de thiazolyle, d'isoxazolyle, de pyridinyle, de pyrimidinyle, de quinoléinyle et d'isoquinoléinyle, où
R4 est hydrogène, 1-4C-alkyle, hydroxy-1-4C-alkyle, 1-4C-alcoxy, 2-4C-alcényloxy, carboxyle, 1-4C-alcoxycarbonyle, carboxy-1-4C-alkyle, halogène, hydroxyle, aryle, aryl-1-4C-alkyle, aryloxy, aryl-1-4C-alcoxy, trifluorométhyle, nitro, amino, mono- ou di-1-4C-alkylamino ou sulfonyle,
R5 est hydrogène, 1-4C-alkyle, 1-4C-alcoxy, 1-4C-alcoxycarbonyle, halogène, trifluorométhyle ou hydroxyle,
R6 est hydrogène, 1-4C-alkyle ou halogène et
R7 est hydrogène, 1-4C-alkyle ou halogène,
où
aryle est phényle ou phényle substitué ayant un, deux ou trois substituants identiques ou différents provenant du groupe constitué de 1-4C-alkyle, de 1-4C-alcoxy, de carboxyle, d'halogène, de trifluorométhyle, de nitro, de trifluorométhoxy, d'hydroxyle et de cyano,
et ses sels.

7. Composé de formule 2a selon la revendication 6, **caractérisé en ce que**
R1 est 1-4C-alkyle ou 3-7C-cycloalkyle,
R2 est hydrogène, 1-4C-alkyle, halogène ou fluoro-1-4C-alkyle,
R3 est halogène, hydroxy-1-4C-alkyle, 1-4C-alcoxy-1-4C-alkyle, 1-4C-alcoxy-1-4C-alcoxy-1-4C-alkyle, 1-4C-alcoxycarbonyle ou le radical -CO-NR31R32,
où
R31 est hydrogène, 1-7C-alkyle, hydroxy-1-4C-alkyle, 3-7C-cycloalkyle ou 1-4C-alcoxy-1-4C-alkyle et
R32 est hydrogène, 1-7C-alkyle, hydroxy-1-4C-alkyle, 3-7C-cycloalkyle ou 1-4C-alcoxy-1-4C-alkyle,
ou où
R31 et R32, conjointement et y compris l'atome d'azote auquel ils sont liés, forment un radical pyrrolidino, pipéridino ou morpholino
Arom est un phényle substitué par R4, R5, R6 et R7,
où
R4 est hydrogène ou 1-4C-alkyle,
R5 est hydrogène ou 1-4C-alkyle,
R6 est hydrogène ou 1-4C-alkyle et
R7 est hydrogène,
et ses sels.

8. Composé de formule 2a selon la revendication 6, **caractérisé en ce que**
R1 est 1-4C-alkyle,
R2 est 1-4C-alkyle,
R3 est halogène, 1-4C-alcoxycarbonyle ou le radical -CO-NR31R32,
où
R31 est hydrogène, 1-4C-alkyle, hydroxy-1-4C-alkyle ou 1-4C-alcoxy-1-4C-alkyle et
R32 est hydrogène, 1-4C-alkyle, hydroxy-1-4C-alkyle ou 1-4C-alcoxy-1-4C-alkyle,
ou où
R31 et R32, conjointement et y compris l'atome d'azote auquel ils sont liés, forment un radical pyrrolidino, pipéridino ou morpholino
Arome est phényle,
et ses sels.

9. Composé de formule 2a 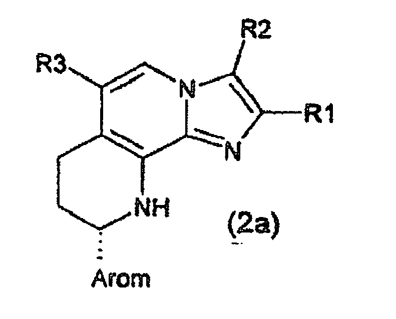 choisi dans le groupe constitué de
(2-méthoxyéthyl)amide d'acide (8S)-2,3-diméthyl-8-phényl-6,7,8,9-tétrahydro-1,3a,9-triazacyclopenta[a]naphtalène-5-carboxylique,
ester éthylique d'acide (8S)-2,3-diméthyl-8-phényl-6,7,8,9-tétrahydro-1,3a,9-triazacyclopenta[a]naphtalène-5-carboxylique,
méthylamide d'acide (8S)-2,3-diméthyl-8-phényl-6,7,8,9-tétrahydro-1,3a,9-triazacyclopenta[a]naphtalène-5-carboxylique,
(2-hydroxyéthyl)amide d'acide (8S)-2,3-diméthyl-8-phényl-6,7,8,9-tétrahydro-1,3a,9-triazacyclopenta[a]naphtalène-5-carboxylique,
diméthylamide d'acide (8S)-2,3-diméthyl-8-phényl-6,7,8,9-tétrahydro-1,3a,9-triazacyclopenta[a]naphtalène-5-carboxylique,
amide d'acide (8S)-2,3-diméthyl-8-phényl-6,7,8,9-tétrahydro-1,3a,9-triazacyclopenta[a]naphtalène-5-carboxylique,
(8S)-1-(2,3-diméthyl-8-phényl-6,7,8,9-tétrahydro-1,3a,9-triazacyclopenta[a]naphtalén-5-yl)-1-morpholin-4-ylméthanone,
(8S)-1-(2,3-diméthyl-8-phényl-6,7,8,9-tétrahydro-1,3a,9-triazacyclopenta[a]naphtalén-5-yl)-1-pyrrolidin-1-ylméthanone,
(8S)-(2,3-diméthyl-8-phényl-6,7,8,9-tétrahydro-1,3a,9-triazacyclopenta[a]naphtalén-5-yl)méthanol,
cyclopropylamide d'acide (8S)-2,3-diméthyl-8-phényl-6,7,8,9-tétrahydro-1,3a,9-triazacyclopenta[a]naphtalène-5-carboxylique,
et ses sels.

10. Procédé de synthèse d'un composé de formule 2, 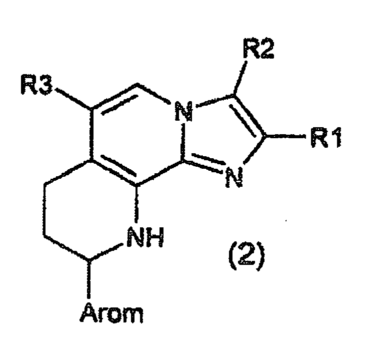 dans laquelle R1, R2, R3 et Arom ont les significations selon la revendication 1, **caractérisé en ce qu'**il comprend la transformation d'un composé de formule 1 selon la revendication 1 en un composé de formule 2, puis, si on le souhaite, la dérivatisation en outre du composé résultant de formule 2 en un autre composé de formule 2.

11. Procédé de synthèse d'un composé de formule 1 selon la revendication 1, **caractérisé en ce qu'**il comprend la transformation d'un composé de formule 5, dans laquelle R1, R2, R3 et Arom ont les significations indiquées dans la revendication 1, en le composé correspondant de formule 1, puis, si on le souhaite, la dérivatisation en outre du composé résultant de formule 1 en un autre composé de formule 1.

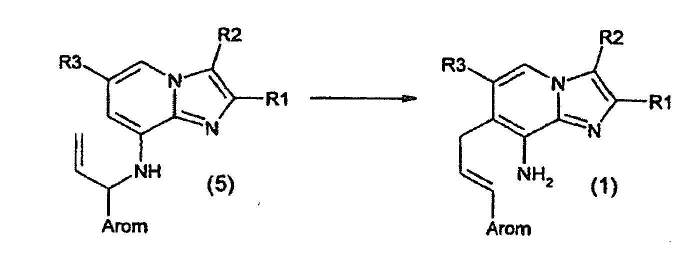

12. Médicament comprenant un composé selon l'une quelconque des revendications 5 à 9 et/ou un sel pharmacologiquement acceptable de celui-ci conjointement aux auxiliaires et/ou excipients pharmaceutiques habituels.

13. Utilisation d'un composé selon l'une quelconque des revendications 5 à 9 et/ou de ses sels pharmacologiquement acceptables pour la production de médicaments destinés à la prévention et au traitement de troubles gastro-intestinaux.
